# EUROPÄISCHE PATENTSCHRIFT

(11) **EP 2 739 611 B1**
(45) Veröffentlichungstag und Bekanntmachung des Hinweises auf die Patenterteilung: **13.05.2015**
(21) Anmeldenummer: 12745447.8
(22) Anmeldetag: 30.07.2012
(51) Int. Cl.: C07D 249/14, C07D 257/06, C07D 401/12, A01N 43/713, A01N 43/653

(54) **N-(TETRAZOL-5-YL)- UND N-(TRIAZOL-5-YL)ARYLCARBONSÄUREAMIDE UND IHRE VERWENDUNG ALS HERBIZIDE**
N-(TETRAZOL-5-YL)- AND N-(TRIAZOL-5-YL)ARYL CARBOXAMIDES AND THEIR USE AS HEBICIDES
CARBOXAMIDES N-(TETRAZOL-5-YL)- ET N-(TRIAZOL-5-YL)ARYL ET LEUR UTILISATION COMME HERBICIDES

(30) Priorität: 03.08.2011 EP 11176378
(43) Veröffentlichungstag der Anmeldung: 11.06.2014
(73) Patentinhaber: Bayer Intellectual Property GmbH, 40789 Monheim (DE)
(72) Erfinder: BRAUN, Ralf, 76857 Ramberg (DE); DÖRNER-RIEPING, Simon, 61267 Neu-Anspach (DE); KÖHN, Arnim, 55270 Klein-Winternheim (DE); AHRENS, Hartmut, 63329 Egelsbach (DE); LEHR, Stefan, 69006 Lyon (FR); VAN ALMSICK, Andreas, 61184 Karben (DE); HÄUSER-HAHN, Isolde, 51375 Leverkusen (DE); DIETRICH, Hansjörg, 65835 Liederbach am Taunus (DE); GATZWEILER, Elmar, 61231 Bad Nauheim (DE); HEINEMANN, Ines, 65719 Hofheim (DE); ROSINGER, Christopher, Hugh, 65719 Hofheim (DE)
(74) Vertreter: BIP Patents
(86) Internationale Anmeldenummer: PCT/EP2012/064863
(87) Internationale Veröffentlichungsnummer: WO 2013/017559

(56) Entgegenhaltungen:
- WO-A1-2004/101532

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2003/010143 und WO2003/010153 sind N-(Tetrazol-5-yl)- und N-(Triazol-5-yl)benzamide und ihre pharmakologische Wirkung bekannt. Aus der prioritätsälteren, nicht vorveröffentlichen EP101748937 sind bestimmte N-(Tetrazol-5-yl)- und N-(Triazol-5-yl)benzamide und -nicotinamide als Herbizide bekannt. Es wurde nun gefunden, dass (Tetrazol-5-yl)- und N-(Triazol-5-yl)arylcarbonsäureamide und -nicotinamide, die in 1-Position des Tetrazol- bzw. Triazolrings spezielle Substituenten tragen, als Herbizide besonders gut geeignet sind.

Ein Gegenstand der vorliegenden Erfindung sind somit N-(Tetrazol-5-yl)- und N-(Triazol-5-yl)arylcarbonsäureamide der Formel (I) oder deren Salze 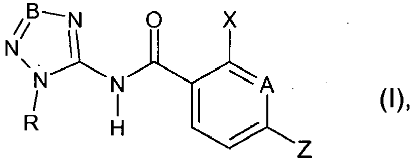 worin
- A: bedeutet N oder CY,
- B: bedeutet N oder CH,
- X: bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-CyCloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
- Y: bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₁-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR'C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO2R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
- Z: bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹ (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)2, (C₁-C₆)-Alkyl-NR¹COR¹, (C₁-C₆)-Alkyl-NR¹SO₂R², N(R¹)₂, P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt, oder Z kann auch Wasserstoff, (C₁-C₆)-Alkyl oder (C₁-C₆)-Alkoxy bedeuten, falls Y für den Rest S(O)ₙR² steht,
- R: bedeutet CH₂R⁶,
durch m Oxogruppen substituiertes CH₂-Heterocyclyl,
durch t (C₁-C₆)-Alkylgruppen substituiertes (C₃-C₇)-Cycloalkyl,
jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Ethyl, Methoxy, Cyano, Nitro, Trifluormethyl und Halogen substituiert sind, und wobei Heterocyclyl und Cycloalkyl n Oxogruppen tragen,
- Q: bedeutet O, S oder NR³,
- R¹: bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO²R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
- R²: bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
- R³: bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
- R⁴: bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
- R⁵: bedeutet (C₁-C₄)-Alkyl,
- R⁶: bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂, Heterocyclyl oder Cycloalkyl, wobei die beiden letztgenannten Reste m Oxo- oder Hydroxygruppen tragen,
- m: bedeutet 1 oder 2,
- n: bedeutet 0, 1 oder 2,
- s: bedeutet 0, 1, 2 oder 3,
- t: bedeutet 1, 2, 3 oder 4,
- u: bedeutet 1, 2, 3, 4 oder 5.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder lod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl. Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so kann diese Gruppe durch ein oder mehrere gleiche oder verschiedene der genannten Reste substituiert sein.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromato-graphische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereo-isomeren und deren Gemische, die von der allgemeinen Formel (I) umfasst, jedoch nicht spezifisch definiert sind.

Bevorzugt sind Verbindungen der allgemeinen Formel (I), worin
- A: bedeutet N oder CY,
- B: bedeutet N oder CH,
- X: bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)2, (C₁-C₆)-Alkyl-SO₂N(R¹)₂, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
- Y: bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, OR¹, COOR¹, OSO₂R², S(O)ₙR², SO₂OR¹, SO₂ N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
- Z: bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder Z kann auch Wasserstoff, (C₁-C₆)-Alkyl oder (C₁-C₆)-Alkoxy bedeuten, falls Y für den Rest S(O)ₙR² steht,
- R: bedeutet CH₂R⁶,
durch m Oxogruppen substituiertes CH₂-Heterocyclyl, jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Ethyl, Methoxy, Cyano, Nitro, Trifluormethyl und Halogen substituiert sind, und wobei Heterocyclyl und Cycloalkyl n Oxogruppen tragen,
- Q: bedeutet O, S oder NR³,
- R¹: bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₂)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
- R²: bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese Reste durch s Reste aus der Gruppe bestehend aus Cyano, Halogen, Nitro, OR³, S(O)ₙR⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
- R³: bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
- R⁴: bedeutet (C₁-C₆)-Alkyl, (C₂-C₆)-Alkenyl oder (C₂-C₆)-Alkinyl,
- R⁵: bedeutet Methyl oder Ethyl,
- R⁶: bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂ oder Heterocyclyl, das m Oxogruppen trägt,
- m: bedeutet 1 oder 2,
- n: bedeutet 0, 1 oder 2,
- s: bedeutet 0, 1, 2 oder 3,
- u: bedeutet 1, 2, 3, 4 oder 5.

Besonders bevorzugt sind Verbindungen der allgemeinen Formel (I), worin
- A: bedeutet N oder CY,
- B: bedeutet N oder CH,
- X: bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
- Y n: Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl Oxogruppen trägt,
- Z: bedeutet Halogen, Cyano, Halogen-(C₁-C₆)-alkyl, (C₃-C₆)-Cycloalkyl, S(O)ₙR², 1,2,4-Triazol-1-yl, oder Z kann auch Wasserstoff, Methyl, Methoxy oder Ethoxy bedeuten, falls Y für den Rest S(O)ₙR² steht,
- R: bedeutet CH₂R⁶, CH₂-Heterocyclyl, wobei Heterocyclyl m Oxogruppen trägt, jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Ethyl, Methoxy, Cyano, Nitro, Trifluormethyl und Halogen substituiert sind, und wobei Heterocyclyl und Cycloalkyl n Oxogruppen tragen,
- Q: bedeutet O, S oder NR³,
- R¹: bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
- R²: bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
- R³: bedeutet Wasserstoff oder (C₁-C₆)-Alkyl,
- R⁴: bedeutet (C₁-C₆)-Alkyl,
- R⁵: bedeutet Methyl oder Ethyl,
- R⁶: bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂,
- m: bedeutet 1 oder 2,
- n: bedeutet 0, 1 oder 2,
- s: bedeutet 0, 1, 2 oder 3,
- u: bedeutet 1, 2, 3, 4 oder 5.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise nach der in Schema 1 angegebenen Methode durch basenkatalysierte Umsetzung eines Benzoesäurechlorids (II) mit einem 5-Amino-1-H-1,2,4-triazol, bzw. 5-Amino-1H-tetrazol (III) hergestellt werden:

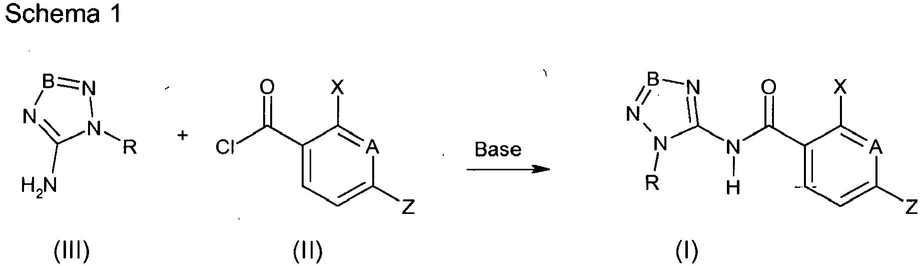

Die Benzoesäurechloride der Formel (II) beziehungsweise die ihnen zugrunde liegenden Benzoesäuren sind grundsätzlich bekannt und können beispielsweise gemäß den in US 6,376,429 B1, EP 1 585 742 A1 und EP 1 202 978 A1 beschriebenen Methoden hergestellt werden.

Erfindungsgemäße Verbindungen können auch nach der in Schema 2 angegebenen Methode durch Umsetzung einer Benzoesäure der Formel (IV) mit einem 5-Amino-1-H-1,2,4-triazol, bzw. 5-Amino-1H-tetrazol (III) hergestellt werden:

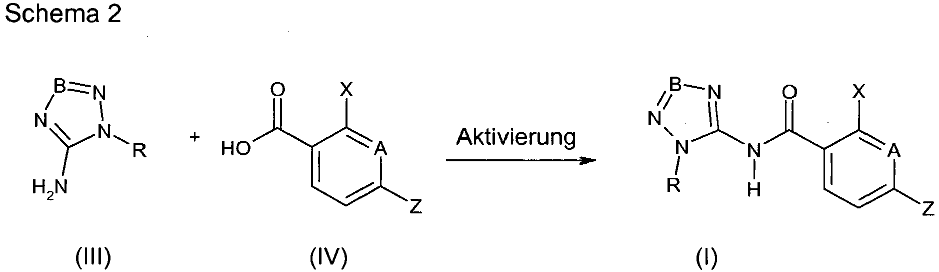

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen können auch nach der in Schema 3 angegebenen Methode durch Umsetzung eines N-(1H-1,2,4-triazol-5-yl)benzamides, N-(1 H-tetrazol-5-yl)benzamides, N-(1H-1,2,4-triazol-5-yl)nicotinamide oder N-(1 H-tetrazol-5-yl)nicotinamide hergestellt werden:

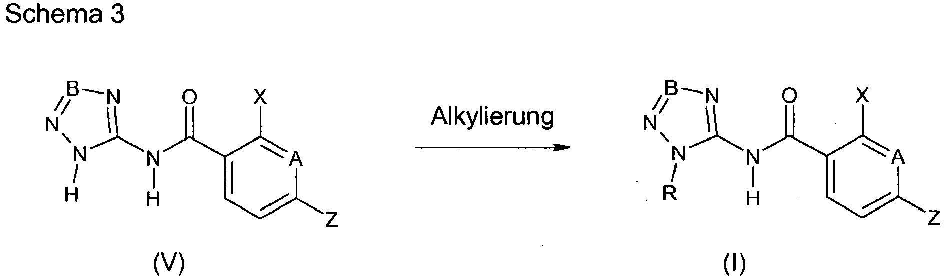

Für diese in Schema 3 genannte Reaktion können Alkylierungsmittel wie Alkylhalogenide, -sulfonate oder Dialkylsulfate in Gegenwart einer Base eingesetzt werden.

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Die 5-Amino-1H-tetrazole der Formel (III) können analog zu literaturbekannten Methoden hergestellt werden. Beispielsweise können 5-Amino-1-R-tetrazole nach der in Journal of the American Chemical Society (1954), 76, 923-924 beschriebenen Methode aus Amino-tetrazol hergestellt werden:

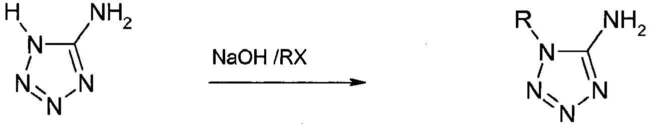

In der oben genannten Formel steht R beispielsweise für einen Alkylrest.

5-Amino-1-R-tetrazole können zum Beispiel wie in Journal of the American Chemical Society (1954) 76, 88-89 beschrieben, synthetisiert werden:

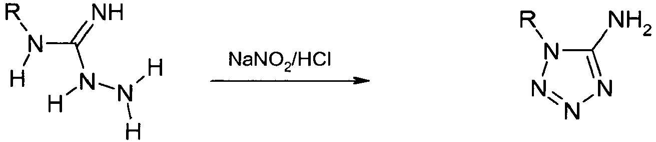

Die 5-Amino-1H-triazole der Formel (III) können analog zu literaturbekannten Methoden hergestellt werden. Beispielsweise können 5-Amino-1-R-triazole nach der Zeitschrift für Chemie (1990), 30(12), 436 - 437 beschriebenen Methode aus Aminotriazol hergestellt werden:

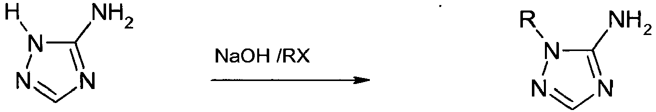

5-Amino-1-R-triazole können auch zum Beispiel wie in Chemische Berichte (1964), 97(2), 396-404 beschrieben, synthetisiert werden:

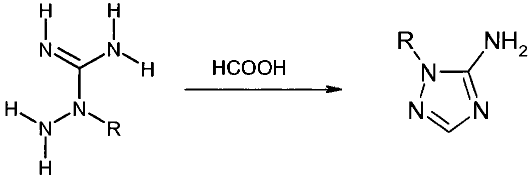

5-Amino-1-R-triazole können auch zum Beispiel wie in Angewandte Chemie (1963), 75, 918 beschrieben, synthetisiert werden:

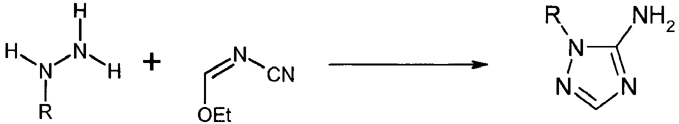

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wichtiger mono-und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer Struktur und der Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf ÖI- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1 H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyrisopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamon, Triallate, Triasulfuron, Triaziflam, Triazofenamide, Tribenuron, Tribenuronmethyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

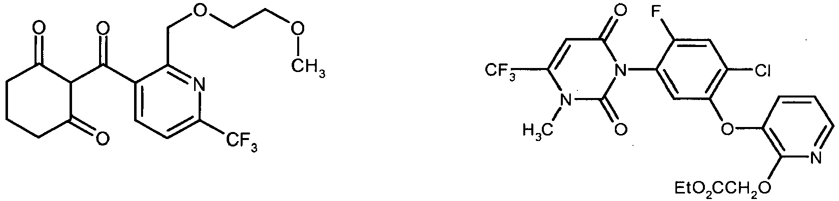

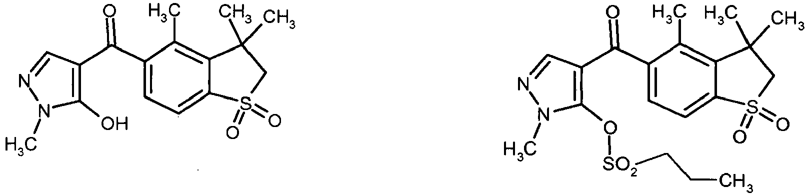

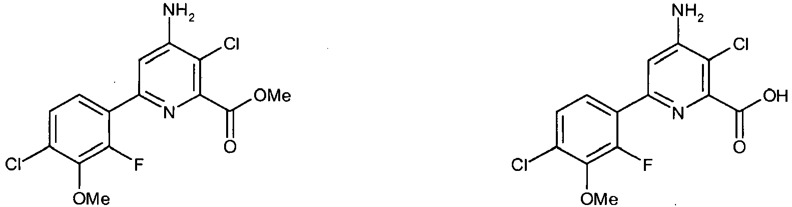

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 2-Chlor-4-(methylsulfonyl)-N-(1-(2-methoxyethyl)-tetrazol-5-yl)-benzamid, (Tabellenbeispiel Nr. 1-12)

176 mg (0.75 mmol) 2-Chlor-4-(methylsulfonyl)-benzoylchlorid, 145 mg (1.0 mmol) 5-Amino-1-(2-methoxyethyl)-tetrazol in 2 ml Pyridin werden 12 h bei 60°C gerührt. Anschließend werden 0.1 ml Wasser zugegeben, 30 min. bei 60°C gerührt und mit EE und 2N HCl versetzt. Die abgetrennte organische Phase wird nochmals mit 2N HCl und Kochsalzlösung gewaschen, über Na₂SO₄ getrocknet, eingedampft und mittels RP-HPLC (Acetonitril/Wassser) gereinigt. Ausbeute 66 mg (23%).

### Synthese von 5-Amino-1-(2-methoxyethyl)-tetrazol

Eine Mischung von 2.33 g (10 mmol) S-Methyl-isothiosemicarbazid-hydroiodid und 751 mg (10 mmol) 2-Methoxyethylamin werden in 10 ml Ethanol solange unter Rückfluß erhitzt, bis kein Methlmercaptan mehr freigesetzt wird. Anschließend wird weitestgehend eingeengt, nacheinander unter Rühren mit 10 ml Wasser, 0.3 ml konz. Salpetersäure und 1.7 g (10 mmol) Silbernitrat in 2 ml Wasser versetzt. Nach 10 min Rühren versetzt man mit 0.5 ml konz. Salzsäure, saugt den Niederschlag ab, wäscht mit 3 ml Wasser und versetzt das Filtrat mit 1.5 ml konz. Salzsäure. Die Mischung wird bei <5°C mit 0.7 g (10 mmol) Natriumnitrit in 2 ml Wasser versetzt und anschließend mit 20 %iger Natronlauge auf pH 10 eingestellt. Anschließend wird 30 min auf 60 °C erhitzt und nach dem Abkühlen mit Essigester extrahiert. Die org. Phase wird 3x mit ges. Kochsalzlösung gewaschen, getrocknet, eingeengt und der Rückstand mit wenig Essigester aufgenommen und filtriert. Beige Kristalle, Ausbeute 590 mg (40%). NMR (DMSO-d₆): 6.62 (brs,2H), 4.26 (t,2H), 3.65 (t,2H), 3.23 (s,3H)

**Tabelle 1: Erfindungsgemäße Verbindungen der Formel (I), worin A für CY, B für N und R für 2-Methoxyethyl steht**

| | | | | |
|---|---|---|---|---|
| 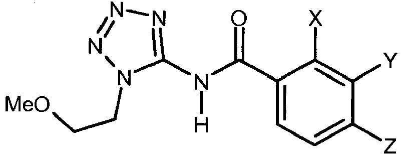 | | | | |

| Nr. | X | Y | Z | Physikalische Daten (¹H-NMR, DMSO-d₆, 400 MHz) |
|---|---|---|---|---|
| 1-1 | F | H | Cl | |
| 1-2 | F | H | Br | |
| 1-3 | F | H | SO₂Me | |
| 1-4 | F | H | SO₂Et | |
| 1-5 | F | H | CF₃ | |
| 1-6 | Cl | H | F | |
| 1-7 | Cl | H | Cl | 8.13 (s,1H), 7.72 (d,1H), 7.61 (dd,1H), 4.55 (t,2H), 3.78 (t,2H), 3.22 (s,3H) |
| 1-8 | Cl | H | Br | 9.90 (bs,1H), 7.69-7.65 (m,2H), 7.49 (dd,1H), 4.63 (t,2H), 3.83 (t,2H), 3.36 (s,3H) |
| 1-9 | Cl | H | I | 9.67 (bs,1H), 7.85 (s,1H), 7.77 (dd,1H), 7.51 (d,1H), 4.60 (t,2H), 3.82 (t,2H), 3.36 (s,3H) |
| 1-10 | Cl | H | SMe | |
| 1-11 | Cl | H | SOMe | |
| 1-12 | Cl | H | SO₂Me | 8.04 (d,1H), 7.98 (dd,1H), 7.83 (s,1H), 4.56 (t,2H), 3.78 (t,2H), 3.37 (s,3H), 3.22 (s,3H) |
| 1-13 | Cl | H | SO₂CH₂Cl | |
| 1-14 | Cl | H | SEt | |
| 1-15 | Cl | H | SO₂Et | |
| 1-16 | Cl | H | CF₃ | 9.84 (bs,1H), 7.90 (d,1H), 7.74 (s,1H), 7.68 (d,1H), 4.62 (t,2H), 3.83 (t,2H), 3.37 (s,3H) |
| 1-17 | Cl | H | CN | |
| 1-18 | Cl | H | NMe2 | |
| 1-19 | Cl | H | NHAc | |
| 1-20 | Cl | H | Pyrrol-1-yl | |
| 1-21 | Cl | H | Pyrrolidin-1-yl | |
| 1-22 | Cl | H | Pyrazol-1-yl | |
| 1-23 | Cl | H | 1,2,4Triazol-1-yl | 9.62 (bs,1H), 8.66 (s, 1H), 8.15 (s,1H), 7.98 (d,1H), 7.92 (s,1H), 7.76 (d,1H), 4.62 (t,2H), 3.84 (t,2H), 3.39 (s,3H) |
| 1-24 | Cl | H | 4-Methyl-3-trifluormethyl-1,2,4-triazolin-5-on-1-yl | |
| 1-25 | Br | H | F | |
| 1-26 | Br | H | Cl | 9.79 (bs,1H), 7.68-7.64 (m,2H), 7.45 (dd,1H), 4.63 (t,2H), 3.83 (t,2H), 3.36 (s,3H) |
| 1-27 | Br | H | Br | |
| 1-28 | Br | H | SMe | 9.8 (bs,1H), 7.65 (d,1H), 7.45 (d,1H), 7.26 (dd,1H), 4.65 (t,2H), 3.84 (t,2H), 3.36 (s,3H), 2.52 (s,3H) |
| 1-29 | Br | H | SO₂Me | |
| 1-30 | Br | H | SO₂Et | |
| 1-31 | Br | H | CF₃ | |
| 1-32 | OMe | H | SO₂Me | |
| 1-33 | SMe | H | F | |
| 1-34 | SMe | H | Cl | 10.65 (bs,1H), 7.83 (d,1H), 7.31 (d,1H), 7.28 (dd,1H), 4.63 (t,2H), 3.83 (t,2H), 3.34 (s,3H), 2.49 (s,3H) |
| 1-35 | SO₂Me | H | Cl | |
| 1-36 | SMe | H | Br | |
| 1-37 | SO₂Me | H | Br | |
| 1-38 | SMe | H | SMe | |
| 1-39 | SO₂Me | H | SMe | |
| 1-40 | SO₂Me | H | SOMe | |
| 1-41 | SO₂Me | H | SO₂Me | |
| 1-42 | SMe | H | CF₃ | 10.6 (bs,1H), 7.95 (d,1H), 7.57 (s,1 H), 7.54 (d,1 H), 4.67 (t,2H), 3.85 (t,2H), 3.34 (s,3H), 2.53 (s,3H) |
| 1-43 | SOMe | H | CF₃ | |
| 1-44 | SO₂Me | H | CF₃ | 8.18 (s,1H), 8.15 (d,1H), 7.97 (d,1H), 4.42 (m,2H), 3.71 (m,2H), 3.54 (s,3H), 3.21 (s,3H) |
| 1-45 | SO₂Et | H | Cl | |
| 1-46 | SO₂Et | H | Br | |
| 1-47 | SO₂Et | H | SMe | |
| 1-48 | SO₂Et | H | SOMe | |
| 1-49 | SO₂Et | H | SO₂Me | |
| 1-50 | SO₂Et | H | CF₃ | |
| 1-51 | SO₂NMePh | H | Cl | |
| 1-52 | SO₂NMe₂ | H | CF₃ | |
| 1-53 | NO₂ | H | F | |
| 1-54 | NO₂ | H | Cl | |
| 1-55 | NO₂ | H | Br | |
| 1-56 | NO₂ | H | I | |
| 1-57 | NO₂ | H | CN | |
| 1-58 | NO₂ | H | SO₂Me | |
| 1-59 | NO₂ | H | SO₂Et | |
| 1-60 | NO₂ | H | CF₃ | 10.3 (bs,1H), 8.44 (s,1H), 8.02 (d,1H), 7.82 (d,1H), 4.63 (t,2H), 3.86 (t,2H), 3.44 (s,3H) |
| 1-61 | Me | H | F | |
| 1-62 | Me | H | OCF₃ | |
| 1-63 | Me | H | Cl | |
| 1-64 | Me | H | Br | |
| 1-65 | Me | H | SO₂Me | |
| 1-66 | Me | H | SO₂CH₂Cl | |
| 1-67 | Me | H | SO₂Et | |
| 1-68 | Me | H | CF₃ | |
| 1-69 | CH₂SMe | H | Br | 10.05 (bs, 1H), 7.58-7.52 (m,3H), 4.65 (t,2H), 3.96 (s,2H), 3.83 (t,2H), 3.34 (s,3H), 2.05 (s,3H) |
| 1-70 | CH₂SO₂Me | H | Br | 9.80 (bs,1H), 7.72-7.68 (m,2H), 7.59 (d,1H), 4.74 (s,2H), 4.62 (t,2H), 3.81 (t,2H), 3.35 (s,3H), 2.98 (s,3H) |
| 1-71 | CH₂SO₂Me | H | CF₃ | |
| 1-72 | Et | H | F | |
| 1-73 | Et | H | Cl | |
| 1-74 | Et | H | Br | |
| 1-75 | Et | H | SO₂Me | |
| 1-76 | Et | H | SO₂CH₂Cl | |
| 1-77 | Et | H | SEt | |
| 1-78 | Et | H | SO₂Et | |
| 1-79 | Et | H | CF₃ | |
| 1-80 | CF₃ | H | Cl | 10.05 (bs,1H), 7.76 (s, 1H), 7.70 (d,1H), 7.67 (d,1H), 4.61 (t,2H), 3.82 (t,2H), 3.33 (s,3H) |
| 1-81 | CF₃ | H | Br | |
| 1-82 | CF₃ | H | SO₂Me | |
| 1-83 | CF₃ | H | SO₂NMe₂ | 10.5 (bs,1H), 8.14 (s,1H), 8.08 (d,1H), 7.89 (d,1H), 4.63 (t,2H), 3.81 (t,2H), 3.34 (s,3H), 2.80 (s,6H) |
| 1-84 | CF₃ | H | CF₃ | |
| 1-85 | NO₂ | NH₂ | F | |
| 1-86 | NO₂ | NHMe | F | |
| 1-87 | NO₂ | NMe₂ | F | |
| 1-88 | NO₂ | Me | Cl | |
| 1-89 | NO₂ | NH₂ | Cl | |
| 1-90 | NO₂ | NHMe | Cl | |
| 1-91 | NO₂ | NMe₂ | Cl | |
| 1-92 | NO₂ | NH₂ | Br | |
| 1-93 | NO₂ | NHMe | Br | |
| 1-94 | NO₂ | NMe₂ | Br | |
| 1-95 | NO₂ | NH₂ | CF₃ | |
| 1-96 | NO₂ | NMe₂ | CF₃ | |
| 1-97 | NO₂ | NH₂ | SO₂Me | |
| 1-98 | NO₂ | NH₂ | SO₂Et | |
| 1-99 | NO₂ | NHMe | SO₂Me | |
| 1-100 | NO₂ | NMe₂ | SO₂Me | |
| 1-101 | NO₂ | NMe2 | SO₂Et | |
| 1-102 | NO₂ | NH₂ | 1H-1,2,4-triazol-1-yl | |
| 1-103 | NO₂ | NHMe | 1H-1,2,4-triazol-1-yl | |
| 1-104 | NO₂ | NMe₂ | 1H-1,2,4-triazol-1-yl | |
| 1-105 | Me | SMe | H | |
| 1-106 | Me | SOMe | H | |
| 1-107 | Me | SO₂Me | H | |
| 1-108 | Me | SEt | H | |
| 1-109 | Me | SOEt | H | |
| 1-110 | Me | SO₂Et | H | |
| 1-111 | Me | S(CH₂)₂OMe | H | |
| 1-112 | Me | SO(CH₂)₂OMe | H | |
| 1-113 | Me | SO₂(CH₂)₂OMe | H | |
| 1-114 | Me | F | F | |
| 1-115 | Me | SEt | F | |
| 1-116 | Me | SOEt | F | |
| 1-117 | Me | SO₂Et | F | |
| 1-118 | Me | Me | Cl | 9.75 (brs, 1H), 7.35 (d,1H), 7.34 (d,1H), 4.62 (t,2H), 3.82 (t,2H), 3.34 (s,3H), 2.46 (s,3H), 2.40 (s,3H) |
| 1-119 | Me | NH₂ | Cl | |

| Nr. | X | Y | Z | Physikalische Daten (¹H-NMR, DMSO-d₆,400 MHz) |
|---|---|---|---|---|
| 1-120 | Me | NHMe | Cl | |
| 1-121 | Me | NMe₂ | Cl | |
| 1-122 | Me | O(CH₂)₂OMe | Cl | |
| 1-123 | Me | O(CH₂)₃OMe | Cl | 9.84 (bs, 1H), 7.35 (d,1H), 7.32 (d,1H), 4.62 (t,2H), 4.00 (t,2H), 3.83 (t,2H), 3.64 (t,2H), 3.38 (s,3H), 3.35 (s,3H), 2.49 (s,3H), 2.11 (m,2H) |
| 1-124 | Me | O(CH₂)₄OMe | Cl | |
| 1-125 | Me | O(CH₂)₂SMe | Cl | 11.45 (bs,1H), 7.51 (d,1H), 7.39 (d,1H), 4.54 (t,2H), 4.07 (t,2H), 3.75 (t,2H), 3.22 (s,3H), 2.92 (t,2H), 2.40 (s,3H), 2.16 (s,3H) |
| 1-126 | Me | O(CH₂)₂SEt | Cl | |
| 1-127 | Me | O(CH₂)₃SMe | Cl | |
| 1-128 | Me | OCH₂CONMe₂ | Cl | 10.27 (brs,1H), 7.39 (d,H), 7.32 (d,1H), 4.63 (t,2H), 4.60 (s,2H), 3.84 (t,2H), 3.35 (s,3H), 3.06 (s,3H), 3.01 (s,3H), 2.41 (s,3H) |
| 1-129 | Me | O(CH₂)₂CONMe₂ | Cl | |
| 1-130 | Me | O(CH₂)₂-NH(CO)NMe₂ | Cl | |
| 1-131 | Me | O(CH₂)₂NH(CO)NHCO₂Et | Cl | |
| 1-132 | Me | O(CH₂)₂NHCO₂Me | Cl | |
| 1-133 | Me | OCH₂NHSO₂cPr | Cl | |
| 1-134 | Me | O(CH₂)-5-(2,4-dimethyl-2,4-dihydro)-3H-1,2,4-triazol-3-on | Cl | |
| 1-135 | Me | O(CH₂)-3,5-dimethyl-1,2-oxazol-4-yl | Cl | |
| 1-136 | Me | F | Cl | |
| 1-137 | Me | Cl | Cl | |
| 1-138 | Me | SMe | Cl | |
| 1-139 | Me | SOMe | Cl | |
| 1-140 | Me | SO₂Me | Cl | |
| 1-141 | Me | SEt | Cl | |
| 1-142 | Me | SOEt | Cl | |
| 1-143 | Me | SO₂Et | Cl | |
| 1-144 | Me | S(CH₂)₂OMe | Cl | |
| 1-145 | Me | SO(CH₂)₂OMe | Cl | |
| 1-146 | Me | SO₂(CH₂)₂OMe | Cl | |
| 1-147 | Me | NH₂ | Br | |
| 1-148 | Me | NHMe | Br | |
| 1-149 | Me | NMe₂ | Br | |
| 1-150 | Me | OMe | Br | 9.94 (bs,1H), 7.53 (d,1H), 7.27 (d,1H), 4.62 (t,2H), 3.85 (s,3H), 3.83 (t,2H), 3.34 (s,3H), 3.22 (s,3H), 2.51 (s,3H) |
| 1-151 | Me | OEt | Br | |
| 1-152 | Me | O(CH₂)₂OMe | Br | 9.86 (bs,1H), 7.52 (d, 1H), 7.25 (d,1H), 4.62 (t,2H), 4.10 (t,2H), 3.84-3.78 (m,4H), 3.47 (s,3H), 3.36 (s,3H), 2.53 (s,3H) |
| 1-153 | Me | O(CH₂)₃OMe | Br | 9.81 (bs,1H), 7.52 (d,1H), 7.24 (d,1H), 4.61 (t,2H), 4.01 (t,2H), 3.83 (t,2H), 3.65 (t,2H), 3.38 (s,3H), 3.35 (s,3H), 2.50 (s,3H), 2.13 (m,2H) |
| 1-154 | Me | O(CH₂)₂SMe | Br | 11.46 (bs,1 H), 7.65 (d,1H), 7.32 (d,1H), 4.54 (t,2H), 4.05 (t,2H), 3.75 (t,2H), 3.22 (s,3H), 2.93 (t,2H), 2.41 (s,3H), 2.17 (s,3H) |
| 1-155 | Me | O(CH₂)₂SEt | Br | 11.45 (bs,1H), 7.65 (d,1H), 7.32 (d,1H), 4.54 (t,2H), 4.03 (t,2H), 3.75 (t,2H), 3.22 (s,3H), 2.96 (t,2H), 2.63 (q,2H), 2.41 (s,3H), 1.22 (t,3H) |
| 1-156 | Me | O(CH₂)₃SMe | Br | |
| 1-157 | Me | OCH₂CONMe₂ | Br | 10.32 (bs,1H), 7.49 (d,1H), 7.32 (d,1H), 4.62 (t,2H), 4.57 (s,2H), 3.83 (t,2H), 3.35 (s,3H), 3.05 (s,3H), 3.01 (s,3H), 2.38 (s,3H) |
| 1-158 | Me | O(CH₂)-5-pyrrolidin-2-on | Br | |
| 1-159 | Me | SMe | Br | |
| 1-160 | Me | SOMe | Br | |
| 1-161 | Me | SO₂Me | Br | |
| 1-162 | Me | SEt | Br | 9.22 (bs,1H), 7.64 (d,1H), 7.28 (d, 1H), 4.60 (t,2H), 3.82 (t,2H), 3.35 (s,3H), 2.88 (q,2H), 2.79 (s,3H), 1.24 (t,3H) |
| 1-163 | Me | SOEt | Br | 9.60 (bs,1H), 7.56 (d,1H), 7.41 (d,1H), 4.60 (t,2H), 3.82 (t,2H), 3.35 (s,3H), 3.29-3.34 (m,1H), 3.10-3.15 (m,1H), 2.82 (s,3H), 1.42 (t,3H) |
| 1-164 | Me | SO₂Et | Br | 9.38 (bs,1H), 7.78 (d,1H), 7.46 (bs,1H), 4.60 (t,2H), 3.82 (t,2H), 3.50 (q,2H), 3.37 (s,3H), 2.85 (s,3H), 1.39 (t,3H) |
| 1-165 | Me | SMe | I | |
| 1-166 | Me | SOMe | I | |
| 1-167 | Me | SO₂Me | I | |
| 1-168 | Me | SEt | I | |

| Nr. | X | Y | Z | Physikalische Daten (¹H-NMR, DMSO-d_{e.}400 MHz) |
|---|---|---|---|---|
| 1-169 | Me | SOEt | I | |
| 1-170 | Me | SO₂Et | I | |
| 1-171 | Me | Cl | CF₃ | 9.95 (brs,1H), 7.68 (d,H), 7.56 (d,1H), 4.64 (t,2H), 3.83 (t,2H), 3.36 (s,3H), 2.59 (s,3H) |
| 1-172 | Me | SMe | CF₃ | 7.74 (d,H), 7.68 (d,1H), 4.48 (m,2H), 3.74 (m,2H), 3.22 (s,3H), 2.70 (s,3H), 2.30 (s,3H) |
| 1-173 | Me | SOMe | CF₃ | 7.90 (s,2H), 4.59 (t,2H), 3.76 (t,2H), 3.24 (s,3H), 3.06 (s,3H), 2.88 (s,3H) |
| 1-174 | Me | SO₂Me | CF₃ | 7.89 (d,1H), 7.82 (d,1H), 4.37 (m,2H), 3.71 (m,2H), 3.37 (s,3H), 3.22 (s,3H), 2.75 (s,3H) |
| 1-175 | Me | SEt | CF₃ | |
| 1-176 | Me | SOEt | CF₃ | |
| 1-177 | Me | SO₂Et | CF₃ | |
| 1-178 | Me | S(CH₂)₂OMe | CF₃ | |
| 1-179 | Me | S(O)(CH₂)₂OMe | CF₃ | |
| 1-180 | Me | SO₂(CH₂)₂OMe | CF₃ | |
| 1-181 | Me | SMe | OMe | |
| 1-182 | Me | SOMe | OMe | |
| 1-183 | Me | SO₂Me | OMe | |
| 1-184 | Me | SEt | OMe | |
| 1-185 | Me | SOEt | OMe | |
| 1-186 | Me | SO₂Et | OMe | |
| 1-187 | Me | Me | SMe | 9.75 (brs,1H), 7.45 (d,1H), 7.08 (d,1H), 4.63 (t,2H), 3.83 (t,2H), 3.35 (s,1H), 2.50 (s,3H), 2.46 (s,3H), 2.32 (s,3H) |
| 1-188 | Me | Me | SO₂Me | 9.90 (brs,1H), 8.02 (d,1H), 7.51 (d,1H), 4.64 (t,2H), 3.83 (t,2H), 3.35 (s,1H), 3.13 (s,3H), 2.70 (s,3H), 2.48 (s,3H) |
| 1-189 | Me | Me | SEt | |
| 1-190 | Me | Me | SO₂Et | |
| 1-191 | Me | 4,5-dihydro-1,2-oxazol-3 yl | SO₂Me | |
| 1-192 | Me | 4,5-dihydro-1,2-oxazol-3 yl | SO₂Et | |
| 1-193 | Me | 5-cyanomethyl- 4,5-dihydro-1,2-oxazol-3-yl | SO₂Me | |
| 1-194 | Me | 5-cyanomethyl- 4,5-dihydro-1,2-oxazol-3-yl | SO₂Et | |
| 1-195 | Me | NH₂ | SO₂Me | |

| Nr. | X | Y | Z | Physikalische Daten (¹H-NMR, DMSO-d₆,400 MHz) |
|---|---|---|---|---|
| 1-196 | Me | NHMe | SO₂Me | |
| 1-197 | Me | NMe₂ | SO₂Me | 9.95 (brs,1H), 7.99 (d,1H), 7.51 (d,1H), 4.64 (t,2H), 3.84 (t,2H), 3.37 (s,1H), 3.27 (s,3H), 2.92 (s,6H), 2.50 (s,3H) |
| 1-198 | Me | NHEt | SO₂Me | |
| 1-199 | Me | NHnPr | SO₂Me | |
| 1-200 | Me | NHiPr | SO₂Me | 9.87 (brs,1H), 7.80 (d,1H), 7.12 (d,1H), 4.64 (t,2H), 3.83 (t,2H), 3.77 (sept,1H), 3.36 (s,1H), 3.11 (s,3H), 2.41 (s,3H), 1.22 (d,6H) |
| 1-201 | Me | NHcPr | SO₂Me | 9.71 (brs,1H), 7.75 (d,1H), 7.06 (d,1H), 6.25 (bs,1H), 4.64 (t,2H), 3.83 (t,2H), 3.36 (s,1H), 3.00 (s,3H), 2.84 (m,1H), 2.60 (s,3H), 0.81 (m,2H), 0.61 (m,2H) |
| 1-202 | Me | NHiBu | SO₂Me | |
| 1-203 | Me | NHCH₂cPr | SO₂Me | |
| 1-204 | Me | NH(CH₂)₂OMe | SO₂Me | 9.95 (bs, 1H), 7.81 (d, 1H), 7.15 (d, 1H), 4.64 (t, 2H), 3.84 (t, 2H), 3.60 (t, 2H), 3.42 (t, 2H), 3.40 (s, 3H), 3.36 (s, 3H), 3.20 (s,3H), 2.43 (s, 3H) |
| 1-205 | Me | NH(CH₂)₂OEt | SO₂Me | |
| 1-206 | Me | NH(CH₂)₃OMe | SO₂Me | |
| 1-207 | Me | NHCH₂CH(OMe)Me | SO₂Me | |
| 1-208 | Me | NHCH₂CH(OMe)CH₂OMe | SO₂Me | |
| 1-209 | Me | NH(CH₂)₃OEt | SO₂Me | |
| 1-210 | Me | NHCH₂-tetrahydrofuran-2-yl | SO₂Me | 9.91 (bs,1H), 7.81 (d,1H), 7.15 (d,1H), 4.64 (t,2H), 4.15 (m,1H), 3.91 (dd,1H), 3.84 (t,2H), 3.79 (dd,1H), 3.43 (dd,1H), 3.36 (s,3H), 3.24 (s,3H), 3.18 (dd,1H), 2.43 (s,3H), 2.09-1.90 (m,3H), 1.72-1.63 (m,1H) |
| 1-211 | Me | NHCH₂-(4-Me-[1,3]dioxolan-2-yl) | SO₂Me | |
| 1-212 | Me | NH(CH₂)₂-(4-Me-[1,3]dioxolan-2-yl) | SO₂Me | |
| 1-213 | Me | NHCH₂-[1,3]dioxan-2-yl | SO₂Me | |
| 1-214 | Me | NHCH₂CONHEt | SO₂Me | |
| 1-215 | Me | Pyrazol-1-yl | SO₂Me | 11.80 (brs,1H), 8.11 (d,1H), 8.01 (d,1H), 7.99 (d,1H), 7.88 (d,1H), 6.59 (t,1H), 4.58 (t,2H), 3.77 (t,2H), 3.22 (s,3H), 3.05 (s,3H), 1.90 (s,3H) |

| Nr. | X | Y | Z | Physikalische Daten (¹H-NMR, DMSO-d_{6,} 400 MHz) |
|---|---|---|---|---|
| 1-216 | Me | 3,5-Me₂-Pyrazol-1-yl | SO₂Me | |
| 1-217 | Me | 1,2,3-Triazol-1-yl | SO₂Me | |
| 1-218 | Me | 1,2,4-Triazol-1-yl | SO₂Me | |
| 1-219 | Me | OH | SO₂Me | |
| 1-220 | Me | OMe | SO₂Me | 10.2 (brs,1H), 7.91 (d,1H), 7.50 (d,1H), 4.65 (t,2H), 3.98 (s,1H), 3.84 (t,2H), 3.37 (s,3H), 3.26 (s,3H), 2.53 (s,3H) |
| 1-221 | Me | OMe | SO₂Et | |
| 1-222 | Me | OEt | SO₂Me | |
| 1-223 | Me | OEt | SO₂Et | |
| 1-224 | Me | OiPr | SO₂Me | |
| 1-225 | Me | OiPr | SO₂Et | |
| 1-226 | Me | O(CH₂)₂OMe | SO₂Me | |
| 1-227 | Me | O(CH₂)₂OMe | SO₂Et | |
| 1-228 | Me | O(CH₂)₃OMe | SO₂Me | |
| 1-229 | Me | O(CH₂)₃OMe | SO₂Et | |
| 1-230 | Me | O(CH₂)₄OMe | SO₂Me | |
| 1-231 | Me | O(CH₂)₄OMe | SO₂Et | |
| 1-232 | Me | O(CH₂)₂SMe | SO₂Me | |
| 1-233 | Me | O(CH₂)₂SEt | SO₂Me | |
| 1-234 | Me | O(CH₂)₃SMe | SO₂Me | |
| 1-235 | Me | O(CH₂)₂NHSO2Me | SO₂Me | |
| 1-236 | Me | O(CH₂)₂NHSO2Me | SO₂Et | |
| 1-237 | Me | OCH₂(CO)NMe₂ | SO₂Me | 10.5 (brs,1H), 7.94 (d,1H), 7.55 (d,1H), 4.82 (s,2H), 4.67 (t,2H), 3.83 (t,2H), 3.40 (s,3H), 3.36 (s,3H), 3.04 (s,3H), 2.95 (s,3H), 2.53 (s,3H) |
| 1-238 | Me | OCH₂(CO)NMe₂ | SO₂Et | |
| 1-239 | Me | OCH₂-tetrahydrofuran-2-yl | SO₂Me | |
| 1-240 | Me | OCH₂-tetrahydrofuran-2-yl | SO₂Et | |
| 1-241 | Me | [1,4]dioxan-2-yl-methoxy | SO₂Me | |
| 1-242 | Me | [1,4]dioxan-2-yl-methoxy | SO₂Et | |
| 1-243 | Me | F | SMe | |
| 1-244 | Me | F | SO₂Me | |
| 1-245 | Me | Cl | SO₂Me | 10.05 (brs,1H), 8.11 (d,H), 7.61 (d,1H), 4.64 (t,2H), 3.84 (t,2H), 3.36 (s,3H), 3.32 (s,3H), 2.61 (s,3H) |
| 1-246 | Me | SMe | SO₂Me | 7.99 (d,1H), 7.74 (d,1H), 4.49 (t,2H), 3.74 (t,2H), 3.52 (s,3H), 3.23 (s,3H), 2.69 (s,3H), 2.38 (s,3H) |
| 1-247 | Me | SOMe | SO₂Me | |
| 1-248 | Me | SO₂Me | SO₂Me | 11.80 (brs,1H), 8.28 (d,1H), 8.08 (d,1H), 4.59 (t,2H), 3.77 (t,2H), 3.60 (s,1H), 3.58 (s,3H), 3.23 (s,3H), 2.72 (s,3H) |
| 1-249 | Me | SO₂Me | SO₂Et | |
| 1-250 | Me | SEt | SO₂Me | |
| 1-251 | Me | SOEt | SO₂Me | |
| 1-252 | Me | SO₂Et | SO₂Me | |
| 1-253 | Me | S(CH₂)₂OMe | SO₂Me | |
| 1-254 | Me | SO(CH₂)₂OMe | SO₂Me | |
| 1-255 | Me | SO₂(CH₂)₂OMe | SO₂Me | |
| 1-256 | CH₂SMe | OMe | SO₂Me | |
| 1-257 | CH₂OMe | OMe | SO₂Me | |
| 1-258 | CH₂O(CH₂)₂O Me | NH(CH₂)₂OEt | SO₂Me | |
| 1-259 | CH₂O(CH₂)₂O Me | NH(CH₂)₃OEt | SO₂Me | |
| 1-260 | CH₂O(CH₂)₃O Me | OMe | SO₂Me | |
| 1-261 | CH₂O(CH₂)₂O Me | NH(CH₂)₂OMe | SO₂Me | |
| 1-262 | CH₂O(CH₂)₂O Me | NH(CH₂)₃OMe | SO₂Me | |
| 1-263 | Et | NH(CH₂)₂OMe | SO₂Me | |
| 1-264 | Et | OMe | SO₂Me | |
| 1-265 | Et | OMe | SO₂Et | |
| 1-266 | Et | OEt | SO₂Me | |
| 1-267 | Et | OEt | SO₂Et | |
| 1-268 | Et | OiPr | SO₂Me | |
| 1-269 | Et | OiPr | SO₂Et | |
| 1-270 | Et | O(CH₂)₂OMe | SO₂Me | |
| 1-271 | Et | O(CH₂)₂OMe | SO₂Et | |
| 1-272 | Et | O(CH₂)₃OMe | SO₂Me | |
| 1-273 | Et | O(CH₂)₃OMe | SO₂Et | |
| 1-274 | Et | F | SO₂Me | |
| 1-275 | Et | SMe | Cl | |
| 1-276 | Et | SOMe | Cl | |
| 1-277 | Et | SO₂Me | Cl | |
| 1-278 | Et | SMe | Br | |
| 1-279 | Et | SOMe | Br | |
| 1-280 | Et | SO₂Me | Br | |
| 1-281 | Et | SMe | I | |
| 1-282 | Et | SOMe | I | |
| 1-283 | Et | SO₂Me | I | |
| 1-284 | Et | SMe | CF₃ | |
| 1-285 | Et | SOMe | CF₃ | |
| 1-286 | Et | SO₂Me | CF₃ | |
| 1-287 | Et | SMe | SO₂Me | |
| 1-288 | Et | SOMe | SO₂Me | |
| 1-289 | Et | SO₂Me | SO₂Me | |
| 1-290 | nPr | SMe | Cl | |
| 1-291 | nPr | SOMe | Cl | |
| 1-292 | nPr | SO₂Me | Cl | |
| 1-293 | nPr | SMe | CF₃ | |
| 1-294 | nPr | SOMe | CF₃ | |
| 1-295 | nPr | SO₂Me | CF₃ | |
| 1-296 | iPr | SMe | Cl | |
| 1-297 | iPr | SOMe | Cl | |
| 1-298 | iPr | SO₂Me | Cl | |
| 1-299 | iPr | SMe | CF₃ | |
| 1-300 | iPr | SOMe | CF₃ | |
| 1-301 | iPr | SO₂Me | CF₃ | |
| 1-302 | cPr | SMe | CF₃ | |
| 1-303 | cPr | SO₂Me | CF₃ | |
| 1-304 | CF₃ | O(CH₂)₂OMe | F | |
| 1-305 | CF₃ | O(CH₂)₃OMe | F | |
| 1-306 | CF₃ | OCH₂CONMe₂ | F | |
| 1-307 | CF₃ | [1,4]dioxan-2-yl-methoxy | F | |
| 1-308 | CF₃ | O(CH₂)₂OMe | Cl | |
| 1-309 | CF₃ | O(CH₂)₃OMe | Cl | |
| 1-310 | CF₃ | OCH₂CONMe₂ | Cl | |
| 1-311 | CF₃ | [1,4]dioxan-2-yl-methoxy | Cl | |
| 1-312 | CF₃ | O(CH₂)₂OMe | Br | |
| 1-313 | CF₃ | O(CH₂)₂OMe | Br | |
| 1-314 | CF₃ | O(CH₂)₃OMe | Br | |
| 1-315 | CF₃ | OCH₂CONMe₂ | Br | |
| 1-316 | CF₃ | [1,4]dioxan-2-yl-methoxy | Br | |
| 1-317 | CF₃ | O(CH₂)₂OMe | I | |
| 1-318 | CF₃ | O(CH₂)₃OMe | I | |
| 1-319 | CF₃ | OCH₂CONMe₂ | I | |
| 1-320 | CF₃ | [1,4]dioxan-2-yl-methoxy | I | |
| 1-321 | CF₃ | F | SO₂Me | |
| 1-322 | CF₃ | F | SO₂Et | |
| 1-323 | CF₃ | O(CH₂)₂OMe | SO₂Me | |
| 1-324 | CF₃ | O(CH₂)₂OMe | SO₂Et | |
| 1-325 | CF₃ | O(CH₂)₃OMe | SO₂Me | |
| 1-326 | CF₃ | O(CH₂)₃OMe | SO₂Et | |
| 1-327 | CF₃ | OCH₂CONMe₂ | SO₂Me | |
| 1-328 | CF₃ | OCH₂CONMe₂ | SO₂Et | |
| 1-329 | CF₃ | [1,4]dioxan-2-yl-methoxy | SO₂Me | |
| 1-330 | CF₃ | [1,4]dioxan-2-yl-methoxy | SO₂Et | |
| 1-331 | F | SMe | CF₃ | |
| 1-332 | F | SOMe | CF₃ | |
| 1-333 | F | SO₂Me | CF₃ | |
| 1-334 | Cl | SMe | H | |
| 1-335 | Cl | SOMe | H | |
| 1-336 | Cl | SO₂Me | H | |
| 1-337 | Cl | SEt | H | |
| 1-338 | Cl | SOEt | H | |
| 1-339 | Cl | SO₂Et | H | |
| 1-340 | Cl | S(CH₂)₂OMe | H | |
| 1-341 | Cl | SO(CH₂)₂OMe | H | |
| 1-342 | Cl | SO₂(CH₂)₂OMe | H | |
| 1-343 | Cl | SMe | Me | 7.40-7.42 (m,1H), 7.08-7.10 (m,1H), 4.30-4.60 (m,2H), 3.70-3.75 (m,2H), 3.35-3.50 (m,2H), 2.54 (s,3H), 2.29 (s,3H) |
| 1-344 | Cl | SOMe | Me | 9.52 (bs,1H), 7.62 (d,1H), 7.30 (d,1H), 4.60 (t,2H), 3.82 (t,2H), 3.37 (s,3H), 2.98 (s,3H), 2.78 (s,3H) |
| 1-345 | Cl | SO₂Me | Me | 9.45 (bs,1H), 7.69 (d, 1H), 7.39 (d,1H), 4.59 (t,2H), 3.82 (t,2H), 3.40 (s,3H), 3.29 (s,3H), 2.83 (s,3H) |
| 1-346 | Cl | SEt | Me | |
| 1-347 | Cl | SOEt | Me | |
| 1-348 | Cl | SO₂Et | Me | |
| 1-349 | Cl | Me | Cl | 9.71 (brs,1H), 7.49 (d,1H), 7.42 (d,1H), 4.62 (t,2H), 3.83 (t,2H), 3.35 (s,3H), 2.53 (s,3H) |
| 1-350 | Cl | CH₂-pyrrolidin-2-on-1-yl | Cl | 9.8 (brs,1H), 7.60 (d,1H), 7.50 (d,1H), 4.83 (s,2H), 4.61 (t,2H), 3.83 (t,2H), 3.38 (s,1H), 3.13 (m,2H), 2.39 (m,2H), 1.96 (m,2H) |
| 1-351 | Cl | CH₂(4-Methyl-3-isopropoxy-1,2,4-triazolin-5-on-1-yl) | Cl | |
| 1-352 | Cl | CH₂(4-Methyl-3-trifluorethoxy-1,2,4-triazolin-5-on-1-yl) | Cl | |
| 1-353 | Cl | NH₂ | Cl | 9.55 (brs,1H), 7.32 (d,1H), 7.01 (d,1H), 4.71 (s,2H), 4.61 (t,2H), 3.82 (t,2H), 3.35 (s,3H) |
| 1-354 | Cl | NHAc | Cl | |
| 1-355 | Cl | NHCON(Me)OMe | Cl | |
| 1-356 | Cl | NHCH₂CONHEt | Cl | |
| 1-357 | Cl | NHCH₂CONHiPr | Cl | |
| 1-358 | Cl | NHCHMeCONHEt | Cl | |
| 1-359 | Cl | imidazolidin-2-on-1-yl | Cl | 11.4 (brs,1H), 7.49 (d,1H), 7.42 (d,1H), 6.02 (brs,1H), 4.64 (t,2H), 3.85-3.68 (m,6H), 3.33 (s,3H) |
| 1-360 | Cl | 1-Methyl-1,2,4-triazolidin-3,5-dion-4-yl) | Cl | 10.6 (brs,1H), 9.8 (brs,1H), 7.70 (d,1H), 7.54 (d,1H), 4.65 (t,2H), 3.83 (t,2H), 3.35 (s,3H), 3.33 (s,3H) |
| 1-361 | Cl | OMe | Cl | 9.85 (bs,1H), 7.48 (d,1H), 7.45 (d,1H), 4.62 (t,2H), 3.94 (s,3H), 3.83 (t,2H), 3.36 (s,3H) |
| 1-362 | Cl | OEt | Cl | 9.90 (bs,1H), 7.46 (d,1H), 7.44 (d,1H), 4.63 (t,2H), 4.13 (q,2H), 3.83 (t,2H), 3.36 (s,3H), 1.48 (t,3H) |
| 1-363 | Cl | OPr | Cl | |
| 1-364 | Cl | OAllyl | Cl | |
| 1-365 | Cl | OCH₂CHF₂ | Cl | |
| 1-366 | Cl | O(CH₂)₂OMe | Cl | 9.32 (bs,1H), 7.47 (d,1H), 7.45 (d,1H), 4.59 (t,2H), 4.21 (t,2H), 3.81 (q,2H), 3.46 (s,3H), 3.36 (s,3H) |
| 1-367 | Cl | O(CH₂)₃OMe | Cl | |
| 1-368 | Cl | OCH₂(CO)NMe₂ | Cl | 9.95 (brs,1H), 7.80 (d,1H), 7.44 (d,1H), 4.68 (s,2H), 4.60 (t,2H), 3.83 (t,2H), 3.36 (s,1H) 3.09 (s,3H), 3.01 (s,3H) |
| 1-369 | Cl | O(CH₂)-5-pyrrolidin-2-on | Cl | |
| 1-370 | Cl | Cl | Cl | 9.95 (brs,1H), 7.58 (d,1H), 7.54 (d,1H), 4.64 (t,2H), 3.84 (t,2H), 3.37 (s,1H) |
| 1-371 | Cl | SMe | Cl | |
| 1-372 | Cl | SOMe | Cl | |
| 1-373 | Cl | SO₂Me | Cl | |
| 1-374 | Cl | SEt | Cl | |
| 1-375 | Cl | SOEt | Cl | |
| 1-376 | Cl | SO₂Et | Cl | |
| 1-377 | Cl | Me | Br | |
| 1-378 | Cl | CH₂(4-Methyl-3-isopropoxy-1,2,4-triazolin-5-on-1-yl) | Br | |
| 1-379 | Cl | CH₂(4-Methyl-3-trifluorethoxy-1,2,4-triazolin-5-on-1-yl) | Br | |
| 1-380 | Cl | NHAc | Br | |
| 1-381 | Cl | NHCON(Me)OMe | Br | |
| 1-382 | Cl | NHCH₂CONHEt | Br | |
| 1-383 | Cl | NHCH₂CONHiPr | Br | |
| 1-384 | Cl | NHCHMeCONHEt | Br | |
| 1-385 | Cl | OMe | Br | 9.49 (bs,1H), 7.62 (d,1H), 7.40 (d,1H), 4.60 (t,2H), 3.92 (s,3H), 3.82 (t,2H), 3.37 (s,3H), |
| 1-386 | Cl | OEt | Br | |
| 1-387 | Cl | OPr | Br | |
| 1-388 | Cl | OAllyl | Br | |
| 1-389 | Cl | OCH₂CHF₂ | Br | |
| 1-390 | Cl | O(CH₂)₂OMe | Br | 11.71 (bs,1H), 7.81 (d,1H), 7.40 (d,1H), 4.56 (t,2H), 4.24 (t,2H), 3.77 (t,2H), 3.74 (t,2H), 3.35 (s,3H), 3.23 (s,3H) |
| 1-391 | Cl | O(CH₂)₃OMe | Br | |
| 1-392 | Cl | OCH₂(CO)NMe₂ | Br | |
| 1-393 | Cl | O(CH₂)-5-pyrrolidin-2-on | Br | |
| 1-394 | Cl | Cl | Br | |
| 1-395 | Cl | SMe | Br | |
| 1-396 | Cl | SOMe | Br | |
| 1-397 | Cl | SO₂Me | Br | |
| 1-398 | Cl | SEt | Br | |
| 1-399 | Cl | SOEt | Br | |
| 1-400 | Cl | SO₂Et | Br | |
| 1-401 | Cl | Me | SMe | |
| 1-402 | Cl | Me | SO₂Me | |
| 1-403 | Cl | Me | SO₂Et | |
| 1-404 | Cl | CO₂H | SO₂Me | |
| 1-405 | Cl | COOMe | SO₂Me | |
| 1-406 | Cl | CONMe₂ | SO₂Me | |
| 1-407 | Cl | CONMe(OMe) | SO₂Me | |
| 1-408 | Cl | CH₂N(OMe)Et | SO₂Me | |
| 1-409 | Cl | CH₂OMe | SO₂Me | |
| 1-410 | Cl | CH₂OMe | SO₂Et | |
| 1-411 | Cl | CH₂OEt | SO₂Me | |
| 1-412 | Cl | CH₂OEt | SO₂Et | |
| 1-413 | Cl | CH₂OiPr | SO₂Me | |
| 1-414 | Cl | CH₂OcPentyl | SO₂Me | |
| 1-415 | Cl | CH₂OCH₂CHF₂ | SO₂Me | |
| 1-416 | Cl | CH₂OCH₂CF₃ | SMe | 9.74 (bs,1H), 7.70 (d,1H), 7.27 (d,1H), 4.99 (s,2H), 4.61 (t,2H), 4.27 (q,2H), 3.72 (t,2H), 3.29 (s,3H), 3.22 (s,3H) |
| 1-417 | Cl | CH₂OCH₂CF₃ | SO₂Me | 7.99 (d,1H), 7.75 (d,1H), 5.24 (s,2H), 4.37 (t,2H), 3.92 (q,2H), 3.81 (t,2H), 3.35 (s,3H), 2.55 (s,3H) |
| 1-418 | Cl | CH₂OCH₂CF₃ | SO₂Et | |
| 1-419 | Cl | CH₂OCH₂CF₂CHF₂ | SO₂Me | |
| 1-420 | Cl | CH₂O(CH₂)₂OMe | SO₂Me | |
| 1-421 | Cl | CH₂PO₃Me₂ | SO₂Me | |
| 1-422 | Cl | CH₂O-tetrahydro-furan-3-yl | SO₂Me | |
| 1-423 | Cl | CH₂O-tetrahydrofuran-3-yl | SO₂Et | |
| 1-424 | Cl | CH₂OCH₂-tetrahydrofuran-2-yl | SO₂Me | 9.65 (bs,1H), 8.18 (d,1H), 7.77 (d,1H), 5.20 (s,2H), 4.61 (t,2H), 4.08 (m,1H), 3.83 (t,2H), 3.82-3.58 (m,4H), 3.37 (s,3H), 3.33 (s,3H), 2.0-1.8 (m,3H), 1.63-1.5 (m,1H), |
| 1-425 | Cl | CH₂OCH₂-tetrahydrofuran-2-yl | SO₂Et | |
| 1-426 | Cl | CH₂OCH₂-tetrahydrofuran-3-yl | SO₂Me | |
| 1-427 | Cl | CH₂OCH₂-tetrahydrofuran-3-yl | SO₂Et | |
| 1-428 | Cl | 4,5-dihydro-1,2-oxazol-3 y | SMe | |
| 1-429 | Cl | 4,5-dihydro-1,2-oxazol-3 yl | SO₂Me | |
| 1-430 | Cl | 4,5-dihydro-1,2-oxazol-3 yl | SO₂Et | |
| 1-431 | Cl | 5-cyanomethyl- 4,5-dihydro-1,2-oxazol-3 yl | SO₂Me | |
| 1-432 | Cl | 5-cyanomethyl- 4,5-dihydro-1,2-oxazol-3 yl | SO₂Et | 10.35 (bs,1H), 8.14 (d,1H), 7.93 (d,1H), 5.16 (m,1H), 4.65 (t,2H), 3.84 (t,2H), 3.73 (dd,1H), 3.38 (s,3H), 3.37 (q,2H), 3.27 (dd,1H), 2.95 (dd, 1H), 2.85 (dd,1H), 1.31 (t,3H) |
| 1-433 | Cl | 5-(MeOCH₂)-4,5-dihydro-1,2-oxazol-3 yl | SO₂Me | |
| 1-434 | Cl | 5-(MeOCH₂)-4,5-dihydro-1,2-oxazol-3 yl | SO₂Et | |
| 1-435 | Cl | 5-Me-5-(MeOCH₂)-4,5-dihydro-1,2-oxazol-3 yl | SO₂Me | |
| 1-436 | Cl | 5-Me-5-(MeOCH₂)-4,5-dihydro-1,2-oxazol-3 yl | SO₂Et | |
| 1-437 | Cl | NH₂ | SO₂Me | |
| 1-438 | Cl | NHMe | SO₂Me | |
| 1-439 | Cl | NMe₂ | SO₂Me | |
| 1-440 | Cl | NH(CH₂)₂OMe | SO₂Me | |
| 1-441 | Cl | NH(CH₂)₂OMe | SO₂Et | |
| 1-442 | Cl | NH(CH₂)₂OEt | SO₂Me | |
| 1-443 | Cl | NH(CH₂)₃OMe | SO₂Me | |
| 1-444 | Cl | NH(CH₂)₃OMe | SO₂Et | |
| 1-445 | Cl | NH(CH₂)₄OMe | SO₂Me | |
| 1-446 | Cl | NH(CH₂)₄OMe | SO₂Et | |
| 1-447 | Cl | pyrazol-1-yl | SO₂Me | |
| 1-448 | Cl | OMe | SO₂Me | |
| 1-449 | Cl | OMe | SO₂Et | |
| 1-450 | Cl | OEt | SO₂Me | 9.41 (bs,1H), 8.02 (d,1H), 7.56 (bd,1H), 4.61 (t,2H), 4.32 (q,2H), 3.84 (t,2H), 3.39 (s,3H), 3.29 (s,3H), 1.42 (t,3H) |
| 1-451 | Cl | OEt | SO₂Et | |
| 1-452 | Cl | OPr | SO₂Me | 9.56 (bs,1H), 8.02 (d,1H), 7.55 (bd,1H), 4.62 (t,2H), 4.22 (t,2H), 3.84 (t,2H), 3.39 (s,3H), 3.28 (s,3H), 1.95 (quin,2H), 1.09 (t,3H) |
| 1-453 | Cl | OPr | SO₂Et | 11.86 (bs,1H), 7.93 (d,1H), 7.71 (d,1H), 4.58 (t,2H), 4.15 (t,2H), 3.78 (t,2H), 3.51 (q,2H), 3.24 (s,3H), 1.88 (sex,2H), 1.13 (t,3H), 1.05 (t,3H) |
| 1-454 | Cl | OiPr | SO₂Me | |
| 1-455 | Cl | OiPr | SO₂Et | |
| 1-456 | Cl | OAllyl | SO₂Me | 9.35 (bs,1H), 8.03 (d,1H), 7.58 (bd,1H), 6.19 (m,1H), 5.51 (dd,1H), 5.37 (dd,1H), 4.75 (d,2H), 4.61 (t,2H), 3.84 (t,2H), 3.40 (s,3H), 3.28 (s,3H) |
| 1-457 | Cl | OAllyl | SO₂Et | |
| 1-458 | Cl | OPropargyl | SO₂Me | 9.63 (bs,1H), 8.03 (d,1H), 7.61 (bd,1H), 4.92 (s,2H), 4.63 (t,2H), 3.84 (t,2H), 3.40 (s,3H), 3.32 (s,3H), 2.66 (t,1H) |
| 1-459 | Cl | OPropargyl | SO₂Et | |
| 1-460 | Cl | O(CH₂)₂F | SO₂Me | 9.50 (bs,1H), 8.04 (d,1H), 7.52 (bd,1H), 3.89-3.91 (m,1H), 4.77-4.79 (m,1H), 4.62 (t,2H), 4.55 (bs,1H), 4.48 (bs,1H), 3.84 (t,2H), 3.40 (s,3H), 3.31 (s,3H) |
| 1-461 | Cl | O(CH₂)₂F | SO₂Et | |
| 1-462 | Cl | O(CH₂)₂Cl | SO₂Me | 9.56 (bs,1H), 8.04 (d,1H), 7.52 (bd,1H), 4.62 (t,2H), 4.51 (t,2H), 3.95 (t,2H), 3.84 (t,2H), 3.41 (s,3H), 3.32 (s,3H) |
| 1-463 | Cl | O(CH₂)₂Cl | SO₂Et | 9.61 (bs,1H), 8.02 (d,1H), 7.60 (bd,1H), 4.62 (t,2H), 4.50 (t,2H), 3.94 (t,2H), 3.84 (t,2H), 3.48 (q,2H), 3.40 (s,3H), 1.28 (t,3H) |
| 1-464 | Cl | OCH₂cPr | SO₂Me | 9.42 (bs,1H), 8.02 (d,1H), 7.55 (d,1H), 4.61 (t,2H), 4.09 (d,2H), 3.83 (t,2H), 3.39 (s,3H), 3.33 (s,3H), 1.40-1.49 (m,1H), 0.65-0.70 (m,2H), 0.45-0.50 (m,2H) |
| 1-465 | Cl | OCH₂cPr | SO₂Et | 9.50 (bs,1H), 8.00 (d,1H), 7.56 (d,1H), 4.62 (t,2H), 4.08 (d,2H), 3.84 (t,2H), 3.51 (q,2H), 3.38 (s,3H), 1.42-1.46 (m,1H), 1.27 (t,3H), 0.65-0.70 (m,2H), 0.44-0.48 (m,2H) |
| 1-466 | Cl | OCH₂cBu | SO₂Me | 9.41 (bs,1H), 8.02 (d,1H), 7.55 (d,1H), 4.62 (t,2H), 4.24 (d,2H), 3.84 (t,2H), 3.39 (s,3H), 3.27 (s,3H), 2.89-2.98 (m,1H), 2.13-2.21 (m,2H), 1.90-2.05 (m,4H) |
| 1-467 | Cl | OCH₂cBu | SO₂Et | |
| 1-468 | Cl | O(CH₂)₂OMe | SO₂Me | 7.76 (d,1H), 7.47 (d,1H), 4.33 (t,2H), 4.27 (t,2H), 3.77 (q,2H), 3.71 (t,2H), 3.36 (s,6H), 3.29 (s,3H) |
| 1-469 | Cl | O(CH₂)₂OMe | SO₂Et | |
| 1-470 | Cl | O(CH₂)₂OEt | SO₂Me | |
| 1-471 | Cl | O(CH₂)₃OMe | SO₂Me | |
| 1-472 | Cl | O(CH₂)₃OMe | SO₂Et | |
| 1-473 | Cl | O(CH₂)₄OMe | SO₂Me | |
| 1-474 | Cl | O(CH₂)₄OMe | SO₂Et | |
| 1-475 | Cl | O(CH₂)₂OCF₃ | SO₂Me | 11.88 (bs,1H), 7.97 (d,1H), 7.75 (d,1 H), 4.57 (q,2H), 4.54-4.56 (m,2H), 4.43-4.48 (m,2H), 3.78 (t,2H), 3.40 (s,3H), 3.24 (s,3H) |
| 1-476 | Cl | O(CH₂)₂OCF₃ | SO₂Et | 11.88 (bs,1H), 7.96 (d,1H), 7.76 (d,1H), 4.58 (t,2H), 4.53-4.56 (m,2H), 4.43-4.46 (m,2H), 3.78 (t,2H), 3.42 (q,2H), 3.24 (s,3H), 1.15 (t,3H) |
| 1-477 | Cl | O(CH₂)₂SMe | SO₂Me | |
| 1-478 | Cl | O(CH₂)₂SEt | SO₂Me | |
| 1-479 | Cl | O(CH₂)₃SMe | SO₂Me | |
| 1-480 | Cl | OCH₂-tetrahydrofuran-2-yl | SO₂Me | |
| 1-481 | Cl | OCH₂-tetrahydrofuran-2-yl | SO₂Et | |
| 1-482 | Cl | [1,4]dioxan-2-yl-methoxy | SO₂Me | |
| 1-483 | Cl | [1,4]dioxan-2-yl-methoxy | SO₂Et | |
| 1-484 | Cl | OCH₂(CO)NMe₂ | SO₂Me | 10.6 (brs,1H), 8.02 (d,1H), 7.63 (d,1H), 4.94 (s,2H), 4.64 (t,2H), 3.84 (t,2H), 3.44 (s,3H), 3.37 (s,3H), 3.03 (s,3H), 2.93 (s,3H) |
| 1-485 | Cl | OCH₂(CO)NMe₂ | SO₂Et | 10.7 (bs,1H), 8.00 (d,1H), 7.63 (d,1H), 4.93 (s,2H), 4.65 (t,2H), 3.83 (t,2H), 3.64 (q,2H), 3.36 (s,3H), 3.02 (s,3H), 2.94 (s,3H), 1.26 (t,3H) |
| 1-486 | Cl | F | SMe | |
| 1-487 | Cl | Cl | SO₂Me | 9.9 (brs,1H), 8.21 (d,1H), 7.74 (d,1H), 4.63 (t,2H), 3.84 (t,2H), 3.39 (s,3H), 3.33 (s,3H) |
| 1-488 | Cl | SMe | SO₂Me | 11.9 (brs,1H), 8.15 (d,1H), 7.94 (d,1H), 4.58 (t,2H), 3.78 (t,2H), 3.59 (s,3H), 3.24 (s,3H), 2.50 (s,3H) |
| 1-489 | Cl | SOMe | SO₂Me | 11.8 (brs,1H), 8.05 (d,1H), 7.89 (d,1 H), 4.42 (t,2H), 3.73 (t,2H), 3.51 (s,3H), 3.22 (s,3H), 3.18 (s,3H), |
| 1-490 | Cl | SO₂Me | SO₂Me | 12.0 (brs,1H), 8.39 (d,1H), 8.27 (d,1H), 4.59 (t,2H), 3.78 (t,2H), 3.67 (s,3H), 3.58 (s,3H), 3.24 (s,3H), |
| 1-491 | Br | OMe | Br | 9.80 (brs,1H), 7.65 (d,1H), 7.30 (d,1H), 4.65 (t,2H), 3.92 (s,3H), 3.84 (t,2H), 3.36 (s,3H) |
| 1-492 | Br | O(CH₂)₂OMe | Br | |
| 1-493 | Br | O(CH₂)₃OMe | Br | |
| 1-494 | Br | OMe | SO₂Me | 9.60 (brs,1H), 7.89 (d,1H), 7.23 (d,1H), 4.66 (t,2H), 4.06 (s,3H), 3.82 (t,2H), 3.41 (s,3H), 3.22 (s,3H) |
| 1-495 | Br | OMe | SO₂Et | |
| 1-496 | Br | OEt | SO₂Me | |
| 1-497 | Br | OEt | SO₂Et | |
| 1-498 | Br | OPr | SO₂Me | |
| 1-499 | Br | OPr | SO₂Et | |
| 1-500 | Br | O(CH₂)₂OMe | SO₂Me | |
| 1-501 | Br | O(CH₂)₂OMe | SO₂Et | |
| 1-502 | Br | O(CH₂)₃OMe | SO₂Me | |
| 1-503 | Br | O(CH₂)₃OMe | SO₂Et | |
| 1-504 | Br | O(CH₂)₄OMe | SO₂Me | |
| 1-505 | Br | O(CH₂)₄OMe | SO₂Et | |
| 1-506 | Br | [1,4]dioxan-2-yl-methoxy | SO₂Me | |
| 1-507 | Br | [1,4]dioxan-2-yl-methoxy | SO₂Et | |
| 1-508 | I | O(CH₂)₂OMe | SO₂Me | |
| 1-509 | I | O(CH₂)₂OMe | SO₂Et | |
| 1-510 | I | O(CH₂)₃OMe | SO₂Me | |
| 1-511 | I | O(CH₂)₃OMe | SO₂Et | |
| 1-512, | I | O(CH₂)₄OMe | SO₂Me | |
| 1-513 | I | O(CH₂)₄OMe | SO₂Et | |
| 1-514 | I | [1,4]dioxan-2-yl-methoxy | SO₂Me | |
| 1-515 | I | [1,4]dioxan-2-yl-methoxy | SO₂Et | |
| 1-516 | OH | SO₂Me | CF₃ | |
| 1-517 | OMe | SMe | CF₃ | 7.76 (d,H), 7.68 (d,1H), 4.53 (t,2H), 3.96 (s,3H), 3.77 (t,2H), 3.24 (s,3H), 2.44 (s,3H) |
| 1-518 | OMe | SOMe | CF₃ | 7.76 (d,1H), 7.68 (d,1H), 4.53 (t,2H), 3.96 (s,3H), 3.77 (t,2H), 3.24 (s,3H), 2.44 (s,3H) |
| 1-519 | OMe | SO₂Me | CF₃ | |
| 1-520 | OMe | SEt | CF₃ | |
| 1-521 | OMe | SOEt | CF₃ | |
| 1-522 | OMe | SO₂Et | CF₃ | |
| 1-523 | OMe | S(CH₂)₂OMe | CF₃ | |
| 1-524 | OMe | SO(CH₂)₂OMe | CF₃ | |
| 1-525 | OMe | SO₂(CH₂)₂OMe | CF₃ | |
| 1-526 | OMe | CH₂N(SO₂Me)Et | Cl | |
| 1-527 | OMe | NHCOMe | Cl | 10.26 (bs,1H), 7.99 (d,1H), 7.38 (d,1H), 4.57 (t,2H), 3.95 (s,3H), 3.80 (t,2H), 3.30 (s,3H), 2.30 (bs,3H) |
| 1-528 | OMe | NHCOEt | Cl | |
| 1-529 | OMe | NHCOiPr | Cl | |
| 1-530 | OMe | NHCOcycPr | Cl | |
| 1-531 | OMe | NHCOCHCMe₂ | Cl | |
| 1-532 | OMe | NHCOPh | Cl | |
| 1-533 | OMe | SMe | Cl | |
| 1-534 | OMe | SOMe | Cl | |
| 1-535 | OMe | SO₂Me | Cl | |
| 1-536 | OMe | SEt | Cl | |
| 1-537 | OMe | SOEt | Cl | |
| 1-538 | OMe | SO₂Et | Cl | |
| 1-539 | OMe | S(CH₂)₂OMe | Cl | |
| 1-540 | OMe | SO(CH₂)₂OMe | Cl | |
| 1-541 | OMe | SO₂(CH₂)₂OMe | Cl | |
| 1-542 | OEt | SMe | Cl | |
| 1-543 | OEt | SOMe | Cl | |
| 1-544 | OEt | SO₂Me | Cl | |
| 1-545 | OCH₂c-Pr | SMe | CF₃ | |
| 1-546 | OCH₂c-Pr | SOMe | CF₃ | |
| 1-547 | OCH₂c-Pr | SO₂Me | CF₃ | |
| 1-548 | OCH₂c-Pr | SEt | CF₃ | |
| 1-549 | OCH₂c-Pr | SOEt | CF₃ | |
| 1-550 | OCH₂c-Pr | SO₂Et | CF₃ | |
| 1-551 | OCH₂c-Pr | S(CH₂)₂OMe | CF₃ | |
| 1-552 | OCH₂c-Pr | SO(CH₂)₂OMe | CF₃ | |
| 1-553 | OCH₂c-Pr | SO₂(CH₂)₂OMe | CF₃ | |
| 1-554 | OCH₂c-Pr | SMe | Cl | |
| 1-555 | OCH₂c-Pr | SOMe | Cl | |
| 1-556 | OCH₂c-Pr | SO₂Me | Cl | |
| 1-557 | OCH₂c-Pr | SEt | Cl | |
| 1-558 | OCH₂c-Pr | SOEt | Cl | |
| 1-559 | OCH₂c-Pr | SO₂Et | Cl | |
| 1-560 | OCH₂c-Pr | S(CH₂)₂OMe | Cl | |
| 1-561 | OCH₂c-Pr | SO(CH₂)₂OMe | Cl | |
| 1-562 | OCH₂c-Pr | SO₂(CH₂)₂OMe | Cl | |
| 1-563 | OCH₂c-Pr | SMe | SO₂Me | |
| 1-564 | OCH₂c-Pr | SOMe | SO₂Me | |
| 1-565 | OCH₂c-Pr | SO₂Me | SO₂Me | |
| 1-566 | OCH₂c-Pr | SEt | SO₂Me | |
| 1-567 | OCH₂c-Pr | SOEt | SO₂Me | |
| 1-568 | OCH₂c-Pr | SO₂Et | SO₂Me | |
| 1-569 | OCH₂c-Pr | S(CH₂)₂OMe | SO₂Me | |
| 1-570 | OCH₂c-Pr | SO(CH₂)₂OMe | SO₂Me | |
| 1-571 | OCH₂c-Pr | SO₂(CH₂)₂OMe | SO₂Me | |
| 1-572 | OSO₂Me | SMe | CF₃ | 11.87 (brs,1H), 7.98 (d,H), 7.94 (d,1H), 4.57 (t,2H), 3.78 (t,2H), 3.61 (s,3H), 3.24 (s,3H), 2.52 (s,3H) |
| 1-573 | OSO₂Me | SOMe | CF₃ | |
| 1-574 | OSO₂Me | SO₂Me | CF₃ | |
| 1-575 | SMe | SMe | H | |
| 1-576 | SO₂Me | SO₂Me | H | |
| 1-577 | SMe | SMe | F | |
| 1-578 | SMe | SEt | F | |
| 1-579 | SO₂Me | NHEt | Cl | |
| 1-580 | SMe | OCH₂CHF₂ | Br | |
| 1-581 | SO₂Me | F | CF₃ | |
| 1-582 | SO₂Me | NH₂ | CF₃ | |
| 1-583 | Cl | SMe | CF₃ | 11.9 (brs,1H), 7.98 (d,1H), 7.93 (d,1H), 4.59 (t,2H), 3.78 (t,2H), 3.24 (s,3H), 2.45 (s,3H) |
| 1-584 | Cl | SOMe | CF₃ | 11.9 (brs,1H), 8.07 (s,2H), 4.59 (t,2H), 3.78 (t,2H), 3.24 (s,3H), 3.16 (s,3H) |
| 1-585 | Cl | SO₂Me | CF₃ | 12.0 (brs,1H), 8.21 (2d, 2H), 4.59 (t,2H), 3.78 (t,2H), 3.54 (s,3H), 3.24 (s,3H) |
| 1-586 | Cl | OCHF₂ | SO₂Me | 10.95 (bs,1H), 8.11 (d,1H) 7.74 (bd,1H), 6.84 (t,1H), 4.64 (t,2H), 3.84 (t,2H), 3.40 (s,3H), 3.28 (s,3H) |
| 1-587 | Cl | OCHF₂ | SO₂Et | 10.05 (bs,1H), 8.09 (d,1H), 7.75 (bd,1H), 6.86 (t,1H), 4.64 (t,2H), 3.84 (t,2H), 3.40 (q,2H), 3.40 (s,3H), 1.30 (t,3H) |
| 1-588 | Me | O(CH₂)₂SOEt | Br | 9.41 (bs,1H), 7.53 (d,1H), 7.23 (d,1H), 4.60 (t,2H), 4.31-4.42 (m,2H), 3.83 (t,2H), 3.37 (s,3H), 3.23-3.30 (m,1H), 3.02-3.09 (m,1H), 2.83-2.94 (m,2H), 2.54 (s,3H), 1.42 (t,3H) |
| 1-589 | Me | O(CH₂)₂SO₂Et | Br | 9.58 (bs,1H), 7.55 (d,1H), 7.24 (d,1H), 4.62 (t,2H), 4.35 (t,2H), 3.83 (t,2H), 3.50 (t,2H), 3.37 (s,3H), 3.29 (q,2H), 2.53 (s,3H), 1.48 (t,3H) |
| 1-590 | Cl | O(CH₂)₂SMe | Cl | 9.71 (bs,1H), 7.47 (s,2H), 4.61 (t,2H), 4.21 (t,2H), 3.82 (t,2H), 3.36 (s,3H), 2.98 (t,2H), 2.22 (s,3H) |
| 1-591 | Et | SEt | CF₃ | 7.70 (d,1H), 7.57 (d,1H), 4.63 (t,2H), 3.83 (t,2H), 3.34 (s,3H), 3.27 (q,2H), 2.80 (q,2H), 1.29-1.23 (m,6H) |
| 1-592 | Et | SOEt | CF₃ | 7.74 - 7.62 (m,2H), 4.62 (t,2H), 3.83 (t,2H), 3.63 (m,1H), 3.52 - 3.25 (m,2H), 3.35 (s,3H), 2.95 (m,1H), 1.46 (t,3H), 1.29 (t,3H) |
| 1-593 | Et | SO₂Et | CF₃ | 7.91 (d,1H), 7.76 (m,1H), 4.61 (t,2H), 3.83 (t,2H), 3.50 - 3.13 (m,7H), 1.50 (t,3H), 1.34 (t,3H) |
| 1-594 | cPr | SOMe | CF₃ | |
| 1-595 | Et | SEt | Cl | |
| 1-596 | Et | SOEt | Cl | |
| 1-597 | Et | SO₂Et | Cl | |
| 1-598 | OMe | SMe | CHF₂ | 8.21 (d,1H), 7.64 (d,1H), 7.23 (t,1H), 4.60 (t,2H), 4.12 (s,3H), 3.82 (t,2H), 3.35 (s,3H), 2.46 (s,3H) in CDCl₃ |
| 1-599 | OMe | SOMe | CHF₂ | |
| 1-600 | OMe | SO₂Me | CHF₂ | |
| 1-601 | OMe | SEt | CHF₂ | |
| 1-602 | OMe | SOEt | CHF₂ | |
| 1-603 | OMe | SO₂Et | CHF₂ | |
| 1-604 | OMe | S(CH₂)₂OMe | CHF₂ | |
| 1-605 | OMe | SO(CH₂)₂OMe | CHF₂ | |
| 1-606 | OMe | SO₂(CH₂)₂OMe | CHF₂ | |
| 1-607 | CH₂OMe | SMe | Cl | |
| 1-608 | CH₂OMe | SOMe | Cl | |
| 1-609 | CH₂OMe | SO₂Me | Cl | |
| 1-610 | CH₂OMe | SEt | Cl | |
| 1-611 | CH₂OMe | SOEt | Cl | |
| 1-612 | CH₂OMe | SO₂Et | Cl | |
| 1-613 | CH₂OMe | S(CH₂)₂OMe | Cl | |
| 1-614 | CH₂OMe | SO(CH₂)₂OMe | Cl | |
| 1-615 | CH₂OMe | SO₂(CH₂)₂OMe | Cl | |
| 1-616 | CH₂OMe | SMe | CF₃ | |
| 1-617 | CH₂OMe | SOMe | CF₃ | |
| 1-618 | CH₂OMe | SO₂Me | CF₃ | |
| 1-619 | CH₂OMe | SEt | CF₃ | |
| 1-620 | CH₂OMe | SOEt | CF₃ | |
| 1-621 | CH₂OMe | SO₂Et | CF₃ | |
| 1-622 | CH₂OMe | S(CH₂)₂OMe | CF₃ | |
| 1-623 | CH₂OMe | SO(CH₂)₂OMe | CF₃ | |
| 1-624 | CH₂OMe | SO₂(CH₂)₂OMe | CF₃ | |
| 1-625 | CH₂OMe | SMe | SO₂Me | |
| 1-626 | CH₂OMe | SOMe | SO₂Me | |
| 1-627 | CH₂OMe | SO₂Me | SO₂Me | |
| 1-628 | CH₂OMe | SEt | SO₂Me | |
| 1-629 | CH₂OMe | SOEt | SO₂Me | |
| 1-630 | CH₂OMe | SO₂Et | SO₂Me | |
| 1-631 | CH₂OMe | S(CH₂)₂OMe | SO₂Me | |
| 1-632 | CH₂OMe | SO(CH₂)₂OMe | SO₂Me | |
| 1-633 | CH₂OMe | SO₂(CH₂)₂OMe | SO₂Me | |
| 1-634 | Et | SEt | SO₂Me | |
| 1-635 | Et | SOEt | SO₂Me | |
| 1-636 | Et | SO₂Et | SO₂Me | |
| 1-637 | Cl | S(CH₂)₂OMe | Cl | |
| 1-638 | Cl | SO(CH₂)₂OMe | Cl | |
| 1-639 | Cl | SO₂(CH₂)₂OMe | Cl | |
| 1-640 | Cl | SEt | SO₂Me | |
| 1-641 | Cl | SOEt | SO₂Me | |
| 1-642 | Cl | SO₂Et | SO₂Me | |
| 1-643 | Cl | S(CH₂)₂OMe | SO₂Me | |
| 1-644 | Cl | SO(CH₂)₂OMe | SO₂Me | |
| 1-645 | Cl | SO₂(CH₂)₂OMe | SO₂Me | |
| 1-646 | Cl | SEt | CF₃ | |
| 1-647 | Cl | SOEt | CF₃ | |
| 1-648 | Cl | SO₂Et | CF₃ | |
| 1-649 | Cl | S(CH₂)₂OMe | CF₃ | |
| 1-650 | Cl | SO(CH₂)₂OMe | CF₃ | |
| 1-651 | Cl | SO₂(CH₂)₂OMe | CF₃ | |

**Tabelle 2: Erfindungsgemäße Verbindungen der Formel (I), worin A für CY steht**

| | | | | | | |
|---|---|---|---|---|---|---|
| 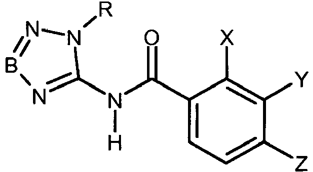 | | | | | | |

| Nr. | B | R | X | Y | Z | Physikalische Daten (¹H-NMR, DMSO-d_{6,} 400 MHz) |
|---|---|---|---|---|---|---|
| 2-1 | N | (CH₂)₂OH | Me | SO₂Me | CF₃ | 7.81 (d,H), 7.72 (d,1H), 4.35 (brs,1H), 4.15 (t,2H), 3.72 (t,2H), 3.35 (s,3H), 2.74 (s,3H) |
| 2-2 | N | (CH₂)₂OEt | Me | SO₂Me | CF₃ | 11.8 (brs,1H), 8.07 (d,H), 8.03 (d,1H), 4.58 (t,2H), 3.79 (t,2H), 3.49 (s,3H), 3.41 (q,2H), 2.76 (s,3H), 1.02 (t,3H) |
| 2-3 | CH | (CH₂)₂OMe | Me | SO₂Me | CF₃ | |
| 2-4 | N | (CH₂)₂OBn | Me | SO₂Me | CF₃ | 7.90 (d,1H), 7.82 (d,1H), 7.34-7.21 (m,5H), 4.52 (t,2H), 4.47 (s,2H), 3.83 (t,2H), 3.39 (s,3H), 2.72 (s,3H) |
| 2-5 | N | CH₂CMe₂OMe | Me | SO₂Me | CF₃ | 11.6 (brs,1H), 7.96 (d,H), 7.84 (d,1H), 4.34 (s,2H), 3.40 (s,3H), 3.13 (s,3H), 2.75 (s,3H), 1.13 (s,6H) |
| 2-6 | N | (CH₂)₃OMe | Me | SO₂Me | CF₃ | 7.97(d,1H), 7.92 (d,1H), 4.30 (m,2H), 3.39 (s,3H), 3.29 (m,2H), 3.21 (s,3H), 2.76 (s,3H), 2.05 (m,2H) |
| 2-7 | N | CH₂CN | Me | SO₂Me | CF₃ | 8.15 (d,1H), 8.11 (d,1H), 5.79 (s,2H), 3.45 (s,3H), 2.75 (s,3H) |
| 2-8 | N | (CH₂)₂NMe₂ | Me | SO₂Me | CF₃ | 7.88 (d,1H), 7.82 (d,1H), 4.27 (m,2H), 3.71 (m,2H), 3.36 (s,3H), 2.75 (m,2H), 2.15 (s,6H) |
| 2-9 | N | CH₂SiMe₃ | Me | SO₂Me | CF₃ | 7.95-7.89 (m,2H), 3.73 (s,2H), 3.39 (s,3H), 2.76 (s,3H), 0.13 (s,9H) |
| 2-10 | N | (CH₂)₂SiMe₃ | | | | |
| 2-11 | N | CH₂PO(OEt)₃ | | | | |
| 2-12 | N | CH₂SMe | | | | |
| 2-13 | N | (CH₂)₂SMe | Me | SO₂Me | CF₃ | 11.9 (brs,1H), 8.06 (s,2H), 4.58 (t,2H), 3.44 (s,3H), 3.02 (t,2H), 2.76 (s,3H), 2.07 (s,3H) |
| 2-14 | N | (CH₂)₂SOMe | Me | SO₂Me | CF₃ | 8.13 (d,1H), 8.06 (d,1H), 4.85-4.78 (m,2H), 3.48-3.38 (m,2H), 3.44 (s,3H), 2.77 (s,3H), 2,63 (s,3H) |
| 2-15 | N | (CH₂)₂SO₂Me | Me | SO₂Me | CF₃ | 7.98 (brs,2H), 4.73 (t,2H), 3.81 (t,2H), 3.40 (s,3H), 3.05 (s,3H), 2.76 (s,3H) |
| 2-16 | N | (CH₂)₂CN | Me | SO₂Me | CF₃ | |
| 2-17 | N | (CH₂)₂NO₂ | Me | SO₂Me | CF₃ | |
| 2-18 | N | CH₂(2-oxopyrrolidin-1-yl) | Me | SO₂Me | CF₃ | |
| 2-19 | N | CH₂CMeOMe | Me | SO₂Me | CF₃ | 11.71 (brs,1H), 8.07 (d,H), 8.00 (d,1H), 4.54 (dd,1H), 4.40 (dd,1H), 3.78 (m,1H), 3.44 (s,3H), 3.16 (s,3H), 2.76 (s,3H), 1.14 (d,3H) |

**Tabelle 3: Erfindungsgemäße Verbindungen der Formel (I), worin A für N steht**

| | | | | | |
|---|---|---|---|---|---|
| 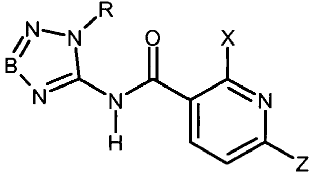 | | | | | |

| Nr. | B | R | X | Z | Physikalische Daten (¹H-NMR, DMSO-d_{6,} 400 MHz) |
|---|---|---|---|---|---|
| 3-1 | CH | C₂H₄OMe | Me | CF₃ | |
| 3-2 | N | C₂H₄OMe | Me | CF₃ | 10.6 (brs,1H), 8.17 (d,1H), 7.68 (d,1 H), 4.69 (t,2H), 3.85 (t,2H), 3.36 (s,3H), 2.84 (s,3H) |
| 3-3 | N | C₂H₄OMe | iPr | CF₃ | |
| 3-4 | CH | C₂H₄OMe | CH₂OMe | CF₃ | |
| 3-5 | N | C₂H₄OMe | CH₂OMe | CF₃ | 11.8 (brs,1H), 8.34 (d,1 H), 8.02 (d,1 H), 4.78 (s,2H), 4.51 (t,2H), 3.77 (t,2H), 3.28 (s,3H), 3.23 (s,3H) |
| 3-6 | N | C₂H₄OMe | CH₂OEt | CF₃ | |
| 3-7 | N | C₂H₄OMe | CH₂OiPr | CF₃ | |
| 3-8 | CH | C₂H₄OMe | CH₂O(CH₂)₂OMe | CF₃ | |
| 3-9 | N | C₂H₄OMe | CH₂O(CH₂)₂OMe | CF₃ | |
| 3-10 | N | C₂H₄OMe | CH₂O(CH₂)₃OMe | CF₃ | |
| 3-11 | N | C₂H₄OMe | CH₂OCH₂CF₃ | CF₃ | |
| 3-12 | N | C₂H₄OMe | CH₂OCH₂cPr | CF₃ | |
| 3-13 | N | C₂H₄OMe | CH₂(3-Me-imidazolin-2-on-1-yl) | CF₃ | |
| 3-14 | N | C₂H₄OMe | CH₂(3-Methoxy-4-methyl-1,2,4-triazolin-5-on-1-yl) | CF₃ | 12.17 (brs,1H), 8.24 (d,1H), 7.77 (d,1H), 5.28 (s,2H), 4.73 (t,2H), 4.05 (s,3H), 3.78 (t,2H), 3.27 (s,3H), 3.12 (s,3H) |
| 3-15 | N | C₂H₄OMe | CH₂SMe | CF₃ | |
| 3-16 | CH | C₂H₄OMe | Cl | CF₃ | |
| 3-17 | N | C₂H₄OMe | Cl | CF₃ | 11.99 (brs,1H), 8.55 (brs,1H), 8.20 (brs,1H), 4.37 (m,2H), 3.72 (m,2H), 2.39 (s,3H) |
| 3-18 | N | C₂H₄OMe | Br | CF₃ | |
| 3-19 | N | C₂H₄OMe | Br | CF₃ | |
| 3-20 | N | C₂H₄OMe | SMe | CF₃ | 8.37 (d,1 H), 7.62 (d,1 H), 4.59 (m,2H), 3.78 (m,2H), 3.23 (s,3H), |
| 3-21 | N | C₂H₄OMe | F | F | |
| 3-22 | N | C₂H₄OMe | F | F | |
| 3-23 | CH | C₂H₄OMe | Cl | Cl | |
| 3-24 | N | C₂H₄OMe | Cl | Cl | 9.95 (bs,1H), 8.16 (d,1H), 7.46 (d,1H), 4.61 (t,2H), 3.83 (t,2H), 3.39 (s,3H) |
| 3-25 | CH | C₂H₄OMe | Me | Cl | |
| 3-26 | N | C₂H₄OMe | Me | Cl | |
| 3-27 | N | C₂H₄OMe | SMe | Cl | |
| 3-28 | N | C₂H₄OMe | SO₂Me | Cl | |
| 3-29 | CH | C₂H₄OMe | Cl | SMe | |
| 3-30 | N | C₂H₄OMe | Cl | SMe | |
| 3-31 | CH | C₂H₄OMe | Me | SO₂Me | |
| 3-32 | N | C₂H₄OMe | Me | SO₂Me | |
| 3-33 | N | C₂H₄OMe | SMe | SMe | |
| 3-34 | N | C₂H₄OMe | SO₂Me | SO₂Me | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
5 Gew.-Teile 2,2'-dinaphthylmethan-6,6'-disulfonsaures Natrium
2 Gew.-Teile oleoylmethyltaurinsaures Natrium,
1 Gew.-Teil Polyvinylalkohol,
17 Gew.-Teile Calciumcarbonat und
50 Gew.-Teile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-16, 1-44, 1-60, 1-125, 1-154, 1-162, 1-163, 1-174, 1-187, 1-191, 1-197, 1-200, 1-204, 1-215, 1-245, 1-246, 1-248, 1-343, 1-345, 1-361, 1-366, 1-368, 1-417, 1-450, 1-452, 1-453, 1-456, 1-458, 1-460, 1-463, 1-464, 1-465, 1-468, 1-475, 1-476, 1-488, 1-494, 1-517, 1-572, 1-583, 2-2, 2-4, 2-5, 2-6, 2-7, 2-19, 3-2 und 3-16 bei einer Aufwandmenge von 320 g/ha jeweils eine mindestens 90%-ige Wirkung gegen Abutilon theophrasti, Amaranthus retroflexus, Matricara inodora, Stellaria media, Veronica persica und Viola tricolor.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-23, 1-44, 1-125, 1-128, 1-154, 1-157, 1-162, 1-188, 1-197, 1-200, 1-215, 1-220, 1-245, 1-246, 1-344, 1-345, 1-350, 1-366, 1-368, 1-417, 1-450, 1-453, 1-456, 1-460, 1-465, 1-468, 1-475, 1-487, 1-488, 1-494, 1-517, 1-518, 1-583, 2-2, 2-4, 2-5, 2-6, 2-19, 3-2, 3-5 und 3-23 bei einer Aufwandmenge von 80 g/ha jeweils eine mindestens 90%-ige Wirkung gegen Abutilon theophrasti, Matricara inodora, Pharbitis purpureum, Stellaria media, Veronica persica und Viola tricolor.

## Patentansprüche

1. N-(Tetrazol-5-yl)- und N-(Triazol-5-yl)arylcarbonsäureamide der Formel (I) oder deren Salze 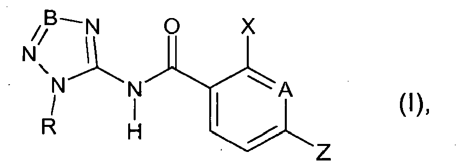 worin
A bedeutet N oder CY,
B bedeutet N oder CH,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO2R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt, oder Z kann auch Wasserstoff, (C₁-C₆)-Alkyl oder (C₁-C₆)-Alkoxy bedeuten, falls Y für den Rest S(O)ₙR² steht,
R bedeutet CH₂R⁶,
durch m Oxogruppen substituiertes CH₂-Heterocyclyl, durch t (C₁-C₆)-Alkylgruppen substituiertes (C₃-C₇)-Cycloalkyl, jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Ethyl, Methoxy, Cyano, Nitro, Trifluormethyl und Halogen substituiert sind, und wobei Heterocyclyl und Cycloalkyl n Oxogruppen tragen,
Q bedeutet O, S oder NR³,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
R⁵ bedeutet (C₁-C₄)-Alkyl,
R⁶ bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂, Heterocyclyl oder Cycloalkyl, wobei die beiden letztgenannten Reste m Oxo- oder Hydroxygruppen tragen,
m bedeutet 1 oder 2,
n bedeutet 0, 1 oder 2,
s bedeutet 0, 1, 2 oder 3,
t bedeutet 1, 2, 3 oder 4,
u bedeutet 1, 2, 3, 4 oder 5.

2. N-(Tetrazol-5-yl)- und N-(Triazol-5-yl)arylcarbonsäureamide nach Anspruch 1, worin
A bedeutet N oder CY,
B bedeutet N oder CH,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)2, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, OR¹, COOR¹, OSO₂R², S(O)ₙR², SO₂OR¹, SO₂ N(R¹)₂, N(R¹)₂, NR¹SO₂R², NR¹COR¹, (C₁-C₆)-Alkyl-S(O)ₙR², (C¹-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder
Z kann auch Wasserstoff, (C₁-C₆)-Alkyl oder (C₁-C₆)-Alkoxy bedeuten, falls Y für den Rest S(O)ₙR² steht,
R bedeutet CH₂R⁶, durch m Oxogruppen substituiertes CH₂-Heterocyclyl, jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Q Ethyl, Methoxy, Cyano, Nitro, Trifluormethyl und Halogen substituiert sind, und wobei Heterocyclyl und Cycloalkyl n Oxogruppen tragen, bedeutet O, S oder NR³,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese Reste durch s Reste aus der Gruppe bestehend aus Cyano, Halogen, Nitro, OR³, S(O)ₙR⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl,
R⁵ bedeutet Methyl oder Ethyl,
R⁶ bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂ oder Heterocyclyl, das m Oxogruppen trägt,
m bedeutet 1 oder 2,
n bedeutet 0, 1 oder 2,
s bedeutet 0, 1, 2 oder 3,
u bedeutet 1, 2, 3, 4 oder 5.

3. N-(Tetrazol-5-yl)- und N-(Triazol-5-yl)arylcarbonsäureamide nach Anspruch 1 oder 2, worin
A bedeutet N oder CY,
B bedeutet N oder CH,
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letztgenannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)_{N}R², SO₂N(R¹)₂, N(R¹)₂, NR¹SO₂R², NR¹COR¹, (C₁-C₆)-Alkyl-S(O)nR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl
n Oxogruppen trägt,
Z bedeutet Halogen, Cyano, Halogen-(C₁-C₆)-alkyl, (C₃-C₆)-Cycloalkyl, S(O)ₙR², 1,2,4-Triazol-1-yl, oder Z kann auch Wasserstoff, Methyl, Methoxy oder Ethoxy bedeuten, falls Y für den Rest S(O)ₙR² steht,
R bedeutet CH₂R⁶, CH₂-Heterocyclyl, wobei Heterocyclyl m Oxogruppen trägt, jeweils durch u Reste aus der Gruppe bestehend aus Nitro, Cyano, Hydroxy, Oxo, SiR⁵₃, PO(OR⁵)₂, S(O)ₙ-(C₁-C₆)-Alkyl, (C₁-C₆)-Alkoxy, Halogen-(C₁-C₆)-alkoxy, N(R³)₂, COR³, OCOR³, NR³COR³, NR³SO₂R⁴, (C₃-C₆)-Cycloalkyl, Heteroaryl, Heterocyclyl, Phenyl, Q-Heteroaryl, Q-Heterocyclyl, Q-Phenyl und Q-Benzyl substituiertes (C₂-C₆)-Alkyl, (C₃-C₇)-Cycloalkyl, Halogen-(C₂-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei die Reste (C₃-C₆)-Cycloalkyl, Heteroaryl, Heterocyclyl, Phenyl, Q-Heteroaryl, Q-Heterocyclyl, Q-Phenyl und Q-Benzyl jeweils durch s Substituenten aus der Gruppe bestehend aus Methyl, Ethyl, Methoxy, Cyano, Nitro, Trifluormethyl und Halogen substituiert sind, und wobei Heterocyclyl und Cycloalkyl n Oxogruppen tragen,
Q bedeutet O, S oder NR³,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
R⁵ bedeutet Methyl oder Ethyl,
R⁶ bedeutet OCOOR⁴, NR⁴COOR⁴, S(O)ₙ-(C₁-C₆)-Alkyl, S(O)ₙ-(C₁-C₆)-Haloalkyl, Nitro, Cyano, SiR⁵₃, PO(OR⁵)₂,
m bedeutet 1 oder 2,
n bedeutet 0, 1 oder 2,
s bedeutet 0, 1, 2 oder 3,
u bedeutet 1, 2, 3, 4 oder 5.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. N-(Tetrazol-5-yl)- and N-(triazol-5-yl)arylcarboxamides of the formula (I) or salts thereof 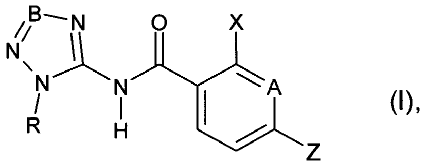 in which
A represents N or CY,
B represents N or CH,
X represents nitro, halogen, cyano, formyl, thiocyanato, (C₁-C₆)-alkyl, halo- (C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR₁R₂, P(O)(OR⁵)₂, CH₂P(O)(OR⁵)₂, (C₁-C₆)-alkyl-heteroaryl, (C₁-C₆)-alkyl-heterocyclyl, where the two last-mentioned radicals are each substituted by s radicals halogen, (C₁-C₆)-alkyl, halo- (C₁-C₆)-alkyl, S(O)ₙ-(C₁-C₆)-alkyl, (C₁-C₆)-alkoxy, halo- (C₁-C₆)-alkoxy, and where heterocyclyl carries n oxo groups,
Y represents hydrogen, nitro, halogen, cyano, thiocyanato, (C₁-C₆)-alkyl, halo- (C₁-C₆)-alkyl, (C₂-C₆)-alkenyl, halo- (C₂-C₆)-alkenyl, (C₂-C₆)-alkynyl, halo-(C₂-C₆)-alkynyl, (C₃-C₆)-cycloalkyl, (C₃-C₆)-cycloalkenyl, halo- (C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-CN, (C₁-C₆)-alkyl-SO₂OR¹, (C₁-C₆)-alkyl-CON(R¹)₂, (C₁-C₆)-alkyl-S02N (R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵), (C₁-C₆)-alkyl-phenyl, (C₁-C₆)-alkyl-heteroaryl, (C₁-C₆)-alkyl-heterocyclyl, phenyl, heteroaryl or heterocyclyl, where the 6 last-mentioned radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo- (C₁-C₆)-alkoxy, (C₁-C₆)-alkoxy-(C₁-C₄)-alkyl and cyanomethyl, and where heterocyclyl carries n oxo groups,
Z represents halogen, cyano, thiocyanato, halo-(C₁-C₆)-alkyl, (C₂-C₆)-alkenyl, halo- (C₂-C₆)-alkenyl, (C₂-C₆)-alkynyl, halo- (C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, heteroaryl, heterocyclyl or phenyl, where the three last-mentioned radicals are each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy and halo- (C₁-C₆)-alkoxy, and where heterocyclyl carries n oxo groups, or Z may also represent hydrogen, (C₁-C₆)-alkyl or (C₁-C₆)-alkoxy if Y represents the radical S(O)ₙR²,
R represents CH₂R⁶, CH₂-heterocyclyl which is substituted by m oxo groups, (C₃-C₇)-cycloalkyl which is substituted by t (C₁-C₆)-alkyl groups,
(C₂-C₆)-alkyl, (C₃-C₇)-cycloalkyl, halo-(C₂-C₆)-alkyl, (C₂-C₆)-alkenyl, halo- (C₂-C₆)-alkenyl, (C₂-C₆)-alkynyl or halo-(C₂-C₆)-alkynyl, each of which is substituted by u radicals from the group consisting of nitro, cyano, hydroxy, oxo, SiR⁵₃, PO(OR⁵)₂, S(O)ₙ-(C₁-C₆)-alkyl, (C₁-C₆)-alkoxy, halo-(C₁-C₆)-alkoxy, N(R³)₂, COR³, OCOR³, NR³COR³, NR³SO₂R⁴, (C₃-C₆)-cycloalkyl, heteroaryl, heterocyclyl, phenyl, Q-heteroaryl, Q-heterocyclyl, Q-phenyl and Q-benzyl, where the radicals (C₃-C₆)-cycloalkyl, heteroaryl, heterocyclyl, phenyl, Q-heteroaryl, Q-heterocyclyl, Q-phenyl and Q-benzyl are each substituted by s substituents from the group consisting of methyl, ethyl, methoxy, cyano, nitro, trifluoromethyl and halogen, and where heterocyclyl and cycloalkyl carry n oxo groups,
Q represents 0, S or NR³,
R¹ represents hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl- (C₁-C₆)-alkyl, phenyl, phenyl-(C₁-C₆)-alkyl, heteroaryl, (C₁-C₆)-alkyl-heteroaryl, heterocycl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 last-mentioned radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy- (C₂-C₆)-alkoxycarbonyl, and where heterocyclyl carries n oxo groups,
R² represents (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 last-mentioned radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)2 and (C₁-C₄)-alkoxy- (C₂-C₆)-alkoxycarbonyl, and where heterocyclyl carries n oxo groups,
R³ represents hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl,
R⁴ represents (C₁-C₆)-alkyl, (C₂-C₆)-alkenyl or (C₂-C₆)-alkynyl, (C₃-C₆)-cycloalkyl or (C₃-C₆)-cycloalkyl-(C₁-C₆)-alkyl,
R⁵ represents (C₁-C₄)-alkyl,
R⁶ represents OCOOR⁴, NR⁴COOR⁴, S (O)ₙ-(C₁-C₆)-alkyl, S(O)ₙ-(C₁-C₆)-haloalkyl, nitro, cyano, SiR⁵₃, PO(OR⁵)₂, heterocyclyl or cycloalkyl, where the two last-mentioned radicals carry m oxo or hydroxy groups,
m represents 1 or 2,
n represents 0, 1 or 2,
s represents 0, 1, 2 or 3,
t represents 1, 2, 3 or 4,
u represents 1, 2, 3, 4 or 5.

2. N-(Tetrazol-5-yl)- and N-(triazol-5-yl)arylcarboxamides according to Claim 1 in which
A represents N or CY,
B represents N or CH,
X represents nitro, halogen, cyano, thiocyanato, (C₁-C₆)-alkyl, halo-(C₁-C₆)-alkyl, (C₂-C₆)-alkenyl, halo- (C₂-C₆)-alkenyl, (C₂-C₆)-alkynyl, halo- (C₃-C₆)-alkynyl, (C₃-C₆)-cycloalkyl, halo- (C₃-C₆)-cycloalkyl, (C₁-C₆)-alkyl-O-(C₁-C₆)-alkyl, (C₃-C₆)-cycloalkyl-(C₁-C₆)-alkyl, halo- (C₃-C₆)-cycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹ or (C₁-C₆)-alkyl-NR¹SO₂R², (C₁-C₆)-alkyl-heteroaryl, (C₁-C₆)-alkyl-heterocyclyl, where the two last-mentioned radicals are each substituted by s radicals halogen, (C₁-C₆)-alkyl, halo- (C₁-C₆)-alkyl, S(O)ₙ-(C₁-C₆)-alkyl, (C₁-C₆)-alkoxy, halo- (C₁-C₆)-alkoxy, and where heterocyclyl carries n oxo groups,
Y represents hydrogen, nitro, halogen, cyano, thiocyanato, (C₁-C₆)-alkyl, halo- (C₁-C₆)-alkyl, (C₂-C₆)-alkenyl, halo- (C₂-C₆)-alkenyl, (C₂-C₆)-alkynyl, halo-(C₃-C₆)-alkynyl, (C₃-C₆)-cycloalkyl, (C₃-C₆)-cycloalkenyl, halo- (C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, OR¹, COOR¹, OSO₂R², S(O)ₙR², SO₂OR¹, SO₂N(R¹)₂, N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², (C₁-C₆)-alkyl-phenyl, (C₁-C₆)-alkyl-heteroaryl, (C₁-C₆)-alkyl-heterocyclyl, phenyl, heteroaryl or heterocyclyl, where the 6 last-mentioned radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo- (C₁-C₆)-alkoxy, (C₁-C₆)-alkoxy-(C₁-C₄)-alkyl and cyanomethyl, and where heterocyclyl carries n oxo groups,
Z represents halogen, cyano, thiocyanato, halo-(C₁-C₆)-alkyl, (C₂-C₆)-alkenyl, halo- (C₂-C₆)-alkenyl, (C₂-C₆)-alkynyl, halo- (C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-alkyl-S (O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², 1,2,4-triazol-1-yl, or
Z may also represent hydrogen, (C₁-C₆)-alkyl or (C₁-C₆)-alkoxy if Y represents the radical S(O)ₙR²,
R represents CH₂R⁶,
CH₂-heterocyclyl which is substituted by m oxo groups, (C₂-C₆)-alkyl, (C₃-C₇)-cycloalkyl, halo- (C₂-C₆)-alkyl, (C₂-C₆)-alkenyl, halo- (C₂-C₆)-alkenyl, (C₂-C₆)-alkynyl or halo-(C₂-C₆)-alkynyl, each of which is substituted by u radicals from the group consisting of nitro, cyano, hydroxy, oxo, SiR⁵₃, PO(OR⁵)₂, S(O)ₙ-(C₁-C₆)-alkyl, (C₁-C₆)-alkoxy, halo-(C₁-C₆)-alkoxy, N(R³)₂, COR³, OCOR³, NR³COR³, NR³SO₂R⁴, (C₃-C₆)-cycloalkyl, heteroaryl, heterocyclyl, phenyl, Q-heteroaryl, Q-heterocyclyl, Q-phenyl and Q-benzyl, where the radicals (C₃-C₆)-cycloalkyl, heteroaryl, heterocyclyl, phenyl, Q-heteroaryl, Q-heterocyclyl, Q-phenyl and Q-benzyl are each substituted by s substituents from the group consisting of methyl, ethyl, methoxy, cyano, nitro, trifluoromethyl and halogen, and where heterocyclyl and cycloalkyl carry n oxo groups,
Q represents 0, S or NR³,
R¹ represents hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where the 16 last-mentioned radicals are substituted by s radicals from the group consisting of cyano, halogen, nitro, OR³, S(O)ₙR⁴, N(R³)₂, NR³OR³, COR³, OCOR³, NR³COR³, NR³SO₂R⁴, CO₂R³, CON(R³)₂ and (C₁-C₄)-alkoxy-(C₂-C₆)-alkoxycarbonyl, and where heterocyclyl carries n oxo groups,
R² represents (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where these radicals are substituted by s radicals from the group consisting of cyano, halogen, nitro, OR³, S(O)ₙR⁴, N(R³)₂, NR³OR³, NR³SO₂R⁴, COR³, OCOR³, NR³COR³, CO₂R³, CON(R³)₂ and (C₁-C₄)-alkoxy-(C₂-C₆)-alkoxycarbonyl, and where heterocyclyl carries n oxo groups, R³ represents hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl,
R⁴ represents (C₁-C₆)-alkyl, (C₂-C₆)-alkenyl or (C₂-C₆)-alkynyl,
R⁵ represents methyl or ethyl,
R⁶ represents OCOOR⁴, NR⁴COOR⁴, S(O)ₙ-(C₁-C₆)-alkyl, S(O)ₙ-(C₁-C₆)-haloalkyl, nitro, cyano, SiR⁵₃, PO(OR⁵)₂ or heterocyclyl which carries m oxo groups,
m represents 1 or 2,
n represents 0, 1 or 2,
s represents 0, 1, 2 or 3,
u represents 1, 2, 3, 4 or 5.

3. N-(Tetrazol-5-yl)- and N-(triazol-5-yl)arylcarboxamides according to Claim 1 or 2 in which
A represents N or CY,
B represents N or CH,
X represents nitro, halogen, cyano, (C₁-C₆)-alkyl, halo-(C₁-C₆)-alkyl, (C₃-C₆)-cycloalkyl, OR¹, S(O)ₙR², (C₁-C₆)-alkyl-S(O)ₙR², (C₁-C₆)-alkyl-OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², (C₁-C₆)-alkyl-heteroaryl, (C₁-C₆)-alkyl-heterocyclyl, where the two last-mentioned radicals are each substituted by s radicals halogen, (C₁-C₆)-alkyl, halo-(C₁-C₆)-alkyl, S(O)ₙ-(C₁-C₆)-alkyl, (C₁-C₆)-alkoxy, halo-(C₁-C₆)-alkoxy, and where heterocyclyl carries n oxo groups,
Y represents hydrogen, nitro, halogen, cyano, (C₁-C₆)-alkyl, (C₁-C₆)-haloalkyl, OR¹, S(O)ₙR², SO₂N(R¹)₂, N(R¹)₂, NR¹SO₂R², NR¹COR¹, (C₁-C₆)-alkyl-S(O)ₙR², (C₁-C₆)-alkyl-OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², (C₁-C₆)-alkyl-phenyl, (C₁-C₆)-alkyl-heteroaryl, (C₁-C₆)-alkyl-heterocyclyl, phenyl, heteroaryl or heterocyclyl, where the 6 last-mentioned radicals are each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy, (C₁-C₆)-alkoxy- (C₁-C₄)-alkyl and cyanomethyl, and where heterocyclyl carries n oxo groups,
Z represents halogen, cyano, halo-(C₁-C₆)-alkyl, (C₃-C₆)-cycloalkyl, S(O)ₙR², 1,2,4-triazol-1-yl, or Z may also represent hydrogen, methyl, methoxy or ethoxy if Y represents the radical S(O)ₙR²,
R represents CH₂R⁶, CH₂-heterocyclyl, where heterocyclyl carries m oxo groups, (C₂-C₆)-alkyl, (C₃-C₇)-cycloalkyl, halo- (C₂-C₆)-alkyl, (C₂-C₆)-alkenyl, halo- (C₂-C₆)-alkenyl, (C₂-C₆)-alkynyl or halo-(C₂-C₆)-alkynyl, each of which is substituted by u radicals from the group consisting of nitro, cyano, hydroxy, oxo, SiR⁵₃, PO(OR⁵)₂, S(O)ₙ-(C₁-C₆)-alkyl, (C₁-C₆)-alkoxy, halo-(C₁-C₆)-alkoxy, N(R³)₂, COR³, OCOR³, NR³COR³, NR³SO₂R⁴, (C₃-C₆)-cycloalkyl, heteroaryl, heterocyclyl, phenyl, Q-heteroaryl, Q-heterocyclyl, Q-phenyl and Q-benzyl, where the radicals (C₃-C₆)-cycloalkyl, heteroaryl, heterocyclyl, phenyl, Q-heteroaryl, Q-heterocyclyl, Q-phenyl and Q-benzyl are each substituted by s substituents from the group consisting of methyl, ethyl, methoxy, cyano, nitro, trifluoromethyl and halogen, and where heterocyclyl and cycloalkyl carry n oxo groups,
Q represents 0, S or NR³,
R¹ represents hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where the 16 last-mentioned radicals are substituted by s radicals from the group consisting of cyano, halogen, nitro, OR³, S(O)ₙR⁴, N(R³)₂, NR³OR³, COR³, OCOR³, NR³COR³, NR³SO₂R⁴, CO₂R³, CON(R³)₂ and (C₁-C₄)-alkoxy-(C₂-C₆)-alkoxycarbonyl, and where heterocyclyl carries n oxo groups,
R² represents (C₁-C₆)-alkyl, (C₃-C₆)-cycloalkyl or (C₃-C₆)-cycloalkyl-(C₁-C₆)-alkyl, where the three radicals mentioned above are each substituted by s radicals from the group consisting of halogen and OR³,
R³ represents hydrogen or (C₁-C₆)-alkyl,
R⁴ represents (C₁-C₆)-alkyl,
R⁵ represents methyl or ethyl,
R⁶ represents OCOOR⁴, NR⁴COOR⁴, S(O)ₙ-(C₁-C₆)-alkyl, S(O)ₙ-(C₁-C₆)-haloalkyl, nitro, cyano, SiR⁵₃, PO(OR⁵)₂,
m represents 1 or 2,
n represents 0, 1 or 2,
s represents 0, 1, 2 or 3,
u represents 1, 2, 3, 4 or 5.

4. Herbicidal compositions **characterized by** a herbicidally effective amount of at least one compound of the formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active compound from the group of insecticides, acaricides, herbicides, fungicides, safeners, and growth regulators.

7. Herbicidal compositions according to Claim 6, comprising a safener.

8. Herbicidal compositions according to Claim 7, comprising cyprosulfamid, cloquintocet-mexyl, mefenpyr-diethyl or isoxadifen-ethyl.

9. Herbicidal compositions according to any of Claims 6 to 8, comprising a further herbicide.

10. Method for controlling unwanted plants, **characterized in that** it comprises applying an effective amount of at least one compound of the formula (I) according to any of Claims 1 to 3 or of a herbicidal composition according to any of Claims 4 to 9 to the plants or the site of the unwanted vegetation.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. Amides d'acides N-(tétrazol-5-yl)- et N-(triazol-5-yl)arylcarboxyliques de formule (I) ou leurs sels 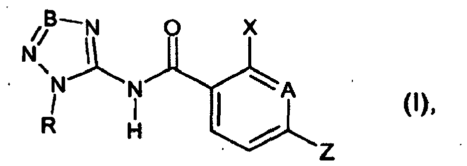 dans lesquels
A signifie N ou CY,
B signifie N ou CH,
X signifie nitro, halogène, cyano, formyle, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², NR₁R₂, P(O)(OR⁵)₂, CH₂P(O)(OR⁵)₂, alkyle en (C₁-C₆)-hétéroaryle, alkyle en (C₁-C₆)-hétérocyclyle, les deux derniers radicaux cités étant chacun substitués par s radicaux halogène, alkyle en (C₁-C₆), halogéno-alkyle en (C₁-C₆), S(O)ₙ-alkyle en (C₁-C₆), alcoxy en (C₁-C₆), halogéno-alcoxy en (C₁-C₆), et l'hétérocyclyle portant n groupes oxo,
Y signifie hydrogène, nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), cycloalcényle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-CN, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², N(R¹)₂, P(O)(OR⁵)₂, CH₂P(O)(OR⁵)₂, alkyle en (C₁-C₆)-phényle, alkyle en (C₁-C₆)-hétéroaryle, alkyle en (C₁-C₆)-hétérocyclyle, phényle, hétéroaryle ou hétérocyclyle, les 6 derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄) et cyanométhyle, et l'hétérocyclyle portant n groupes oxo,
Z signifie halogène, cyano, rhodano, halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², N(R¹)₂, P(O)(OR⁵)₂, hétéroaryle, hétérocyclyle ou phényle, les trois derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆)ou halogéno-alcoxy en (C₁-C₆), et l'hétérocyclyle portant n groupes oxo, ou Z peut également signifier hydrogène, alkyle en (C₁-C₆) ou alcoxy en (C₁-C₆)si Y représente le radical S(O)ₙR²,
R signifie CH₂R⁶, CH₂-hétérocyclyle substitué par m groupes oxo, cycloalkyle en (C₃-C₇) substitué par t groupes alkyle en (C₁-C₆),
alkyle en (C₂-C₆), cycloalkyle en (C₃-C₇), halogéno-alkyle en (C₂-C₆), alcényle en (C₂-C₆), halogéno-alcényle en (C₂-C₆), alcynyle en (C₂-C₆) ou halogéno-alcynyle en (C₂-C₆), chacun substitué par u radicaux du groupe constitué par nitro, cyano, hydroxy, oxo, SiR⁵₃, PO(OR⁵)₂, S(O)ₙ-alkyle en (C₁-C₆), alcoxy en (C₁-C₆), halogéno-alcoxy en (C₁-C₆), N(R³)₂, COR³, OCOR³, NR³COR³, NR³SO₂R⁴, cycloalkyle en (C₃-C₆), hétéroaryle, hétérocyclyle, phényle, Q-hétéroaryle, Q-hétérocyclyle, Q-phényle et Q-benzyle, les radicaux cycloalkyle en (C₃-C₆), hétéroaryle, hétérocyclyle, phényle, Q-hétéroaryle, Q-hétérocyclyle, Q-phényle et Q-benzyle étant chacun substitués par s substituants du groupe constitué par méthyle, éthyle, méthoxy, cyano, nitro, trifluorométhyle et halogène, et l'hétérocyclyle et le cycloalkyle portant n groupes oxo,
Q signifie 0, S ou NR³,
R¹ signifie hydrogène, alkyle en (C₁-C₆), halogénoalkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et alcoxy en (C₁-C₄)-alcoxycarbonyle en (C₂-C₆), et l'hétérocyclyle portant n groupes oxo,
R² signifie alkyle en (C₁-C₆), halogénoalkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)2 et alcoxy en (C₁-C₄)-alcoxycarbonyle en (C₂-C₆), et l'hétérocyclyle portant n groupes oxo,
R³ signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆),
R⁴ signifie alkyle en (C₁-C₆), alcényle en (C₂-C₆) ou alcynyle en (C₂-C₆), cycloalkyle en (C₃-C₆) ou cycloalkyle en (C₃-C₆)-alkyle en (C₁-C₆),
R⁵ signifie alkyle en (C₁-C₄),
R⁶ signifie OCOOR⁴, NR⁴COOR⁴, S(O)ₙ-alkyle en (C₁-C₆), S(O)ₙ-haloalkyle en (C₁-C₆), nitro, cyano, SiR⁵₃, PO(OR⁵)₂, hétérocyclyle ou cycloalkyle, les deux derniers radicaux cités portant m groupes oxo ou hydroxy,
m signifie 1 ou 2,
n signifie 0, 1 ou 2,
s signifie 0, 1, 2 ou 3,
t signifie 1, 2, 3 ou 4,
u signifie 1, 2, 3, 4 ou 5.

2. Amides d'acides N-(tétrazol-5-yl)- et N-(triazol-5-yl)arylcarboxyliques selon la revendication 1, dans lesquels
A signifie N ou CY,
B signifie N ou CH,
X signifie nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), alkyle en (C₁-C₆)-O-alkyle en (C₁-C₆), cycloalkyle en (C₃-C₆)-alkyle en (C₁-C₆), halogéno-cycloalkyle en (C₃-C₆)-alkyle en (C₁-C₆), COR¹, OR¹, OCOR¹, OSO₂R², S(O)ₙR², SO₂OR¹, SO₂N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹ ou alkyle en (C₁-C₆)-NR¹SO₂R², alkyle en (C₁-C₆)-hétéroaryle, alkyle en (C₁-C₆)-hétérocyclyle, les deux derniers radicaux cités étant chacun substitués par s radicaux halogène, alkyle en (C₁-C₆), halogéno-alkyle en (C₁-C₆), S(O)ₙ-alkyle en (C₁-C₆), alcoxy en (C₁-C₆), halogéno-alcoxy en (C₁-C₆), et l'hétérocyclyle portant n groupes oxo,
Y signifie hydrogène, nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), cycloalcényle en (C₃-C₆), halogéno-cycloalkyle en (C₃-C₆), cycloalkyle en (C₃-C₆)-n alkyle en (C₁-C₆), halogéno-cycloalkyle en (C₃-C₆)-alkyle en (C₁-C₆), COR¹, OR¹, COOR¹, OSO₂R², S(O)ₙR², SO₂OR¹, SO₂N(R¹)₂, N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², alkyle en (C₁-C₆)-phényle, alkyle en (C₁-C₆)-hétéroaryle, alkyle en (C₁-C₆)-hétérocyclyle, phényle, hétéroaryle ou hétérocyclyle, les six derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄) et cyanométhyle, et l'hétérocyclyle portant groupes oxo,
Z signifie halogène, cyano, rhodano, halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², 1,2,4-triazol-1-yle, ou
Z peut également signifier hydrogène, alkyle en (C₁-C₆) ou alcoxy en (C₁-C₆)si Y représente le radical S(O)ₙR²,
R signifie CH₂R⁶, CH₂-hétérocyclyle substitué par m groupes oxo,
alkyle en (C₂-C₆), cycloalkyle en (C₃-C₇), halogéno-alkyle en (C₂-C₆), alcényle en (C₂-C₆), halogéno-alcényle en (C₂-C₆), alcynyle en (C₂-C₆) ou halogéno-alcynyle en (C₂-C₆), chacun substitué par u radicaux du groupe constitué par nitro, cyano, hydroxy, oxo, SiR⁵₃, PO(OR⁵)₂, S(O)ₙ-alkyle en (C₁-C₆), alcoxy en (C₁-C₆), halogéno-alcoxy en (C₁-C₆), N(R³)₂, COR³, OCOR³, NR³COR³, NR³SO₂R⁴, cycloalkyle en (C₃-C₆), hétéroaryle, hétérocyclyle, phényle, Q-hétéroaryle, Q-hétérocyclyle, Q-phényle et Q-benzyle, les radicaux cycloalkyle en (C₃-C₆), hétéroaryle, hétérocyclyle, phényle, Q-hétéroaryle, Q-hétérocyclyle, Q-phényle et Q-benzyle étant chacun substitués par s substituants du groupe constitué par méthyle, éthyle, méthoxy, cyano, nitro, trifluorométhyle et halogène, et l'hétérocyclyle et le cycloalkyle portant n groupes oxo,
Q signifie 0, S ou NR³,
R¹ signifie hydrogène, alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆), alkyle en (C₁-C₆)-O-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle ou alkyle en (C₁-C₆)-NR³-hétérocyclyle, les 16 derniers radicaux cités étant substitués par s radicaux du groupe constitué par cyano, halogène, nitro, OR³, S(O)ₙR⁴, N(R³)₂, NR³OR³, COR³, OCOR³, NR³COR³, NR³SO₂R⁴, CO₂R³, CON(R³)₂ et alcoxy en (C₁-C₄) -alcoxycarbonyle en (C₂-C₆), et l'hétérocyclyle portant n groupes oxo,
R² signifie alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆), alkyle en (C₁-C₆)-O-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆) -NR³-hétéroaryle ou alkyle en (C₁-C₆) -NR³-hétérocyclyle, ces radicaux étant substitués par s radicaux du groupe constitué par cyano, halogène, nitro, OR³, S(O)ₙR⁴, N(R³)₂, NR³OR³, NR³SO₂R⁴, COR³, OCOR³, NR³COR³, CO₂R³, CON(R³)₂ et alcoxy en (C₁-C₄)-alcoxycarbonyle en (C₂-C₆), et l'hétérocyclyle portant n groupes oxo,
R³ signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆),
R⁴ signifie alkyle en (C₁-C₆), alcényle en (C₂-C₆) ou alcynyle en (C₂-C₆),
R⁵ signifie méthyle ou éthyle,
R⁶ signifie OCOOR⁴, NR⁴COOR⁴, S(O)ₙ-alkyle en (C₁-C₆), S(O)ₙ-haloalkyle en (C₁-C₆), nitro, cyano, SiR⁵₃, PO(OR⁵)₂ ou hétérocyclyle, qui porte m groupes oxo,
m signifie 1 ou 2,
n signifie 0, 1 ou 2,
s signifie 0, 1, 2 ou 3,
u signifie 1, 2, 3, 4 ou 5.

3. Amides d'acides N-(tétrazol-5-yl)- et N-(triazol-5-yl)arylcarboxyliques selon la revendication 1 ou 2, dans lesquels
A signifie N ou CY,
B signifie N ou CH,
X signifie nitro, halogène, cyano, alkyle en (C₁-C₆), halogéno-alkyle en (C₁-C₆), cycloalkyle en (C₃-C₆), OR¹, S(O)ₙR², alkyle en (C₁-C₆)-S(O)ₙR², alkyle en (C₁-C₆)-OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², alkyle en (C₁-C₆)-hétéroaryle, alkyle en (C₁-C₆)-hétérocyclyle, les deux derniers radicaux cités étant chacun substitués par s radicaux halogène, alkyle en (C₁-C₆), halogéno-alkyle en (C₁-C₆), S(O)ₙ-alkyle en (C₁-C₆), alcoxy en (C₁-C₆), halogéno-alcoxy en (C₁-C₆), et l'hétérocyclyle portant n groupes oxo,
Y signifie hydrogène, nitro, halogène, cyano, alkyle en (C₁-C₆), halogéno-alkyle en (C₁-C₆), OR¹, S(O)ₙR², SO₂N(R¹)₂, N(R¹)₂, NR¹SO₂R², NR¹COR¹, alkyle en (C₁-C₆)-S(O)ₙR², alkyle en (C₁-C₆)-OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², alkyle en (C₁-C₆)-phényle, alkyle en (C₁-C₆)-hétéroaryle, alkyle en (C₁-C₆)-hétérocyclyle, phényle, hétéroaryle ou Z hétérocyclyle, les 6 derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄) et cyanométhyle, et l'hétérocyclyle portant n groupes oxo, signifie halogène, cyano, halogéno-alkyle en (C₁-C₆), cycloalkyle en (C₃-C₆), S(O)ₙR², 1,2,4-triazol-1-yle, ou Z peut également signifier hydrogène, méthyle, méthoxy ou éthoxy si Y représente le radical S(O)ₙR²,
R signifie CH₂R⁶, CH₂-hétérocyclyle, hétérocyclyle portant m groupes oxo, alkyle en (C₂-C₆), cycloalkyle en (C₃-C₇), halogéno-alkyle en (C₂-C₆), alcényle en (C₂-C₆), halogéno-alcényle en (C₂-C₆), alcynyle en (C₂-C₆) ou halogéno-alcynyle en (C₂-C₆), chacun substitué par u radicaux du groupe constitué par nitro, cyano, hydroxy, oxo, SiR⁵₃, PO(OR⁵)₂, S(O)ₙ-alkyle en (C₁-C₆), alcoxy en (C₁-C₆), halogéno-alcoxy en (C₁-C₆), N(R³)₂, COR³, OCOR³, NR³COR³, NR³SO₂R⁴, cycloalkyle en (C₃-C₆), hétéroaryle, hétérocyclyle, phényle, Q-hétéroaryle, Q-hétérocyclyle, Q-phényle et Q-benzyle, les radicaux cycloalkyle en (C₃-C₆), hétéroaryle, hétérocyclyle, phényle, Q-hétéroaryle, Q-hétérocyclyle, Q-phényle et Q-benzyle étant chacun substitués par s substituants du groupe constitué par méthyle, éthyle, méthoxy, cyano, nitro, trifluorométhyle et halogène, et l'hétérocyclyle et le cycloalkyle portant n groupes oxo,
Q signifie 0, S ou NR³,
R¹ signifie hydrogène, alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆), alkyle en (C₁-C₆)-O-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle ou alkyle en (C₁-C₆)-NR³-hétérocyclyle, les 16 derniers radicaux cités étant substitués par s radicaux du groupe constitué par cyano, halogène, nitro, OR³, S(O)ₙR⁴, N(R³)₂, NR³OR³, COR³, OCOR³, NR³COR³, NR³SO₂R⁴, CO₂R³, CON(R³)₂ et alcoxy en (C₁-C₄)-alcoxycarbonyle en (C₂-C₆), et l'hétérocyclyle portant n groupes oxo,
R² signifie alkyle en (C₁-C₆), cycloalkyle en (C₃-C₆) ou cycloalkyle en (C₃-C₆)-alkyle en (C₁-C₆), les trois derniers radicaux étant chacun substitués par s radicaux du groupe constitué par halogène et OR³,
R³ signifie hydrogène ou alkyle en (C₁-C₆),
R⁴ signifie alkyle en (C₁-C₆),
R⁵ signifie méthyle ou éthyle,
R⁶ signifie OCOOR⁴, NR⁴COOR⁴, S(O)ₙ-alkyle en (C₁-C₆), S(O)ₙ-haloalkyle en (C₁-C₆), nitro, cyano, SiR⁵₃, PO(OR⁵)₂,
m signifie 1 ou 2,
n signifie 0, 1 ou 2,
s signifie 0, 1, 2 ou 3,
u signifie 1, 2, 3, 4 ou 5.

4. Agents herbicides, **caractérisés par** une teneur herbicide efficace en au moins un composé de formule (I) selon l'une quelconque des revendications 1 à 3.

5. Agents herbicides selon la revendication 4, en mélange avec des adjuvants de formulation.

6. Agents herbicides selon la revendication 4 ou 5, contenant au moins une autre substance à effet pesticide du groupe constitué par les insecticides, les acaricides, les herbicides, les fongicides, les agents protecteurs et les régulateurs de croissance.

7. Agents herbicides selon la revendication 6, contenant un agent protecteur.

8. Agents herbicides selon la revendication 7, contenant du cyprosulfamide, du cloquintocet-mexyl, du méfenpyr-diéthyle ou de l'isoxadifen-éthyle.

9. Agents herbicides selon l'une quelconque des revendications 6 à 8, contenant un herbicide supplémentaire.

10. Procédé de lutte contre les plantes indésirables, **caractérisé en ce qu'**une quantité efficace d'au moins un composé de formule (I) selon l'une quelconque des revendications 1 à 3 ou d'un agent herbicide selon l'une quelconque des revendications 4 à 9 est appliquée sur les plantes ou à l'emplacement de la végétation indésirable.

11. Utilisation de composés de formule (I) selon l'une quelconque des revendications 1 à 3 ou d'agents herbicides selon l'une quelconque des revendications 4 à 9 pour lutter contre les plantes indésirables.

12. Utilisation selon la revendication 11, **caractérisée en ce que** les composés de formule (I) sont utilisés pour lutter contre les plantes indésirables dans les cultures de plantes utiles.

13. Utilisation selon la revendication 12, **caractérisée en ce que** les plantes utiles sont des plantes utiles transgéniques.
